(19) Europäisches Patentamt
European Patent Office
Office européen des brevets

(11) **EP 3 441 738 A2**

(12) **EUROPEAN PATENT APPLICATION**

(43) Date of publication:
**13.02.2019 Bulletin 2019/07**

(51) Int Cl.:
***G01L 1/20*** (2006.01)

(21) Application number: **18183658.6**

(22) Date of filing: **16.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.07.2017 US 201715651260**

(71) Applicant: **Aurora Flight Sciences Corporation Manassas, VA 20110 (US)**

(72) Inventors:
• **KREBS, Christopher**
  **Manassas, VA 20110 (US)**
• **CHAMBERS, Jeffrey**
  **Manassas, VA 20110 (US)**
• **OPPERMAN, Roedolph A.**
  **Manassas, VA 20110 (US)**
• **FAIR, David**
  **Marlborough, MA 01752 (US)**

(74) Representative: **Morrall, Jonathan Ian McLachlan et al**
**Kilburn & Strode LLP**
**Lacon London**
**84 Theobalds Road**
**London WC1X 8NL (GB)**

(54) **DYNAMIC LOAD SENSOR FOR MICROGRAVITY**

(57)    Disclosed herein is a dynamic load sensor system for use in microgravity environments. The dynamic load sensor system may include a first sensor pad unit, a second sensor pad unit, processing unit, and an operator interface module. Each of the first sensor pad unit and the second sensor pad unit may include a sensor base plate, a top plate, and a plurality of load cells positioned therebetween. The processing unit may include a processor operatively coupled with an internal memory device, while the operator interface module includes a display device and a plurality of operator input devices.

**EP 3 441 738 A2**

Figure 2a

**Description**

## STATEMENT OF GOVERNMENT INTEREST

This invention was made with government support under Contract Number: NNX14CS55C awarded by the National Aeronautics and Space Administration (NASA). The government has certain rights in the invention.

## TECHNICAL FIELD

The present disclosure relates to systems, methods, and apparatuses to facilitate collection and analysis of biomechanical data in microgravity environments.

## BACKGROUND

Exercise countermeasures are used extensively onboard the International Space Station to mitigate bone mineral density loss and muscle atrophy among crewmembers during long duration spaceflight missions. Exercising in space poses unique challenges, but without exercise, astronauts can lose up to 15% of their muscle mass, some of it can be lost permanently. The advanced resistive exercise device (ARED) uses a piston and flywheel system to simulate free-weight exercise routines in normal gravity to work all the major muscle groups through squats, dead lifts, and calf raises. The ARED was transported to the International Space Station on space shuttle mission STS-126 in November 2008. ARED users can achieve results similar to those from free-weight training, suggesting that it could be an effective countermeasure against loss of conditioning during spaceflight. Indeed, resistive exercise is a countermeasure that prevents the major muscle groups from weakening and lessens bone loss. While the ARED's primary goal is to maintain muscle strength and mass, resistive exercise also helps astronauts increase endurance for physically demanding tasks such as space walks.

These exercise countermeasure systems are generally effective, but currently do not include on-orbit collection and analysis of biomechanical data that may advance the efficiency of these systems in mitigating the incidence of bone and muscle loss. Despite current countermeasures, bone losses on International Space Station have generally been 1-2% per month. By consistently measuring foot forces on the ARED, while also tracking astronaut bone health, adjustments can be made (*e.g.*, modified weightlifting protocols) to improve effectiveness in bone loss mitigation. To that end, the subject disclosure provides systems, methods, and apparatuses to facilitate collection and analysis of, *inter alia,* biomechanical data.

## SUMMARY

The present disclosure is directed to systems, methods, and apparatuses to facilitate collection and analysis of, *inter alia,* biomechanical data.

According to a first aspect, a dynamic load sensor system for use in microgravity environments comprises: a first sensor pad unit and a second sensor pad unit, wherein each of the first sensor pad unit and the second sensor pad unit comprises a sensor base plate, a top plate, and a plurality of load cells positioned between the sensor base plate and the top plate; a processing unit having a processor operatively coupled with an internal memory device; and an operator interface module having a display device and a plurality of operator input devices.

In certain aspects, each of the plurality of load cells comprises twelve foil strain gages.

In certain aspects, each of the plurality of load cells comprises three full Wheatstone strain gage bridges.

In certain aspects, the operator interface module enables an operator to navigate between a plurality of operational modes using one or more of the plurality of operator input devices.

In certain aspects, the plurality of operational modes includes a standby mode, one or more work out modes, a download mode, and an upload mode.

In certain aspects, the one or more work out modes includes a set up mode and a run mode.

In certain aspects, each of the first sensor pad unit and the second sensor pad unit are configured to non-invasively couple with a preexisting exercise device.

In certain aspects, the preexisting exercise device is a resistive exercise device.

In certain aspects, each of the processing unit, the operator interface module, the first sensor pad unit, and the second sensor pad unit are separate components, and the processing unit is operatively coupled with the operator interface module, the first sensor pad unit, and the second sensor pad unit via a plurality of interconnecting cables.

In certain aspects, at least one of the processing unit or the operator interface module includes a data port communicatively coupled with the processor, the data port being configured to removably couple with an external memory device.

In certain aspects, the top plate comprises a first surface residing in a first plane and a second surface residing in a second plane that is substantially parallel to the first plane, wherein the first surface is shaped to include a geometric pattern.

In certain aspects, the geometric pattern is a triangular pattern.

In certain aspects, the first surface includes a network of ribs and spars to reinforce a least a portion of a perimeter of the top plate.

In certain aspects, the second surface includes a gripping material.

In certain aspects, the second surface includes a plurality of bench leg holes/slots to secure an exercise bench.

In certain aspects, the sensor base plate comprises a first surface residing in a first plane and a second surface residing in a second plane that is substantially parallel to the first plane, wherein the first surface includes a plurality of sensor recesses to secure the plurality of load cells.

In certain aspects, the first surface further comprises a hard-stop rim along a least a portion of a perimeter of the sensor base plate to prohibit the top plate from compressing in the event of overloading via the top plate, thereby mitigating damage to the load cells.

According to a second aspect, a method for performing load sensor calibration of a sensor pad unit in a dynamic load sensor system, where the sensor pad unit having a plurality of load cells positioned between a sensor base plate and a top plate, comprises: determining a first calibration coefficient for a first load cell having a first rotation angle, and a second calibration coefficient for a second load cell having a second rotation angle; converting a first local load cell voltage from the first load cell to a first local reaction force using the first calibration coefficient, and a second local load cell voltage from the second load cell to a second local reaction force using the second calibration coefficient; determining a first rotation matrix for the first load cell as a function of the first rotation angle, and a second rotation matrix for the second load cell as a function of the second rotation angle; transforming the first local reaction force from a local coordinate to a global coordinate as a function of the first rotation matrix to yield a first global reaction force, and the second local reaction force from a local coordinate to a global coordinate as a function of the second rotation matrix to yield a second global reaction force; determining a summation matrix to sum the first global reaction force and the second global reaction force; and summing the first global reaction force and the second global reaction force using the summation matrix to provide global reaction forces and moments for the sensor pad unit.

In certain aspects, the method further comprises the steps of: determining a third calibration coefficient for a third load cell having a third rotation angle, and a fourth calibration coefficient for a fourth load cell having a fourth rotation angle; converting a third local load cell voltage from the third load cell to a third local reaction force using the third calibration coefficient, and a fourth local load cell voltage from the fourth load cell to a fourth local reaction force using the fourth calibration coefficient; determining a third rotation matrix for the third load cell as a function of the third rotation angle, and a fourth rotation matrix for the fourth load cell as a function of the fourth rotation angle; and transforming the third local reaction force from a local coordinate to a global coordinate as a function of the third rotation matrix to yield a third global reaction force, and the fourth local reaction force from a local coordinate to a global coordinate as a function of the fourth rotation matrix to yield a fourth global reaction force, wherein the global reaction forces and moments for the sensor pad unit is a summation of the first, second, third, and fourth global reaction forces.

In certain aspects, each of the first, second, third, and fourth calibration coefficients is determined experimentally.

In certain aspects, each of the plurality of load cells comprises twelve foil strain gages.

In certain aspects, each of the plurality of load cells comprises three full Wheatstone strain gage bridges.

An embodiment comprises a dynamic load sensor system for use in microgravity environments, the dynamic load sensor system may include a first sensor pad unit and a second sensor pad unit, wherein each of the first sensor pad unit and the second sensor pad unit may include a sensor base plate, a top plate, and a plurality of load cells positioned between the sensor base plate and the top plate; a processing unit having a processor operatively coupled with an internal memory device; and an operator interface module having a display device and a plurality of operator input devices. At least one of the plurality of load cells may include twelve foil strain gages. At least one of the plurality of load cells may include three full Wheatstone strain gage bridges. The operator interface module may enable an operator to navigate between a plurality of operational modes using one or more of the plurality of operator input devices. The plurality of operational modes may include a standby mode, one or more work out modes, a download mode, and an upload mode. The one or more work out modes may include a set up mode and a run mode. At least one of the first sensor pad unit and the second sensor pad unit may be configured to non-invasively couple with a preexisting exercise device. The preexisting exercise device may be a resistive exercise device. At least one of the processing unit, the operator interface module, the first sensor pad unit, and the second sensor pad unit may be separate components, and the processing unit may be operatively coupled with the operator interface module, the first sensor pad unit, and the second sensor pad unit via a plurality of interconnecting cables. At least one of the processing unit or the operator interface module may include a data port communicatively coupled with the processor, the data port being configured to removably couple with an external memory device. The top plate may include a first surface residing in a first plane and a second surface residing in a second plane that may be substantially parallel to the first plane, wherein the first surface may be shaped to include a geometric pattern. The geometric pattern may be a triangular pattern. The first surface may include a network of ribs and spars to reinforce a least a portion of a perimeter of the top plate. The second surface may include a gripping material. The second surface may include a plurality of bench leg holes/slots to secure an exercise bench. The sensor base plate may include a first surface residing in a first plane and a second surface residing in a second plane that may be substantially parallel to the first plane, wherein the first surface may include a plurality of sensor recesses to secure the plurality of load cells. The first surface further may include a hard-stop rim along a least a portion of a perimeter of the sensor base plate to prohibit the top plate from compressing in the event of overloading via the top plate, thereby mitigating damage to the load cells.

Another embodiment comprises a method for performing load sensor calibration of a sensor pad unit in a dynamic load sensor system, the sensor pad unit having a plurality of load cells positioned between a sensor base plate and a top plate, the method includes determining a first calibration coefficient for a first load cell having a first rotation angle, and a second calibration coefficient for a second load cell having a second rotation angle; converting a first local load cell voltage from the first load cell to a first local reaction force using the first calibration coefficient, and a second local load cell voltage from the second load cell to a second local reaction force using the second calibration coefficient; determining a first rotation matrix for the first load cell as a function of the first rotation angle, and a second rotation matrix for the second load cell as a function of the second rotation angle; transforming the first local reaction force from a local coordinate to a global coordinate as a function of the first rotation matrix to yield a first global reaction force, and the second local reaction force from a local coordinate to a global coordinate as a function of the second rotation matrix to yield a second global reaction force; determining a summation matrix to sum the first global reaction force and the second global reaction force; and summing the first global reaction force and the second global reaction force using the summation matrix to provide global reaction forces and moments for the sensor pad unit. The method may also include the steps of: determining a third calibration coefficient for a third load cell having a third rotation angle, and a fourth calibration coefficient for a fourth load cell having a fourth rotation angle; converting a third local load cell voltage from the third load cell to a third local reaction force using the third calibration coefficient, and a fourth local load cell voltage from the fourth load cell to a fourth local reaction force using the fourth calibration coefficient; determining a third rotation matrix for the third load cell as a function of the third rotation angle, and a fourth rotation matrix for the fourth load cell as a function of the fourth rotation angle; and transforming the third local reaction force from a local coordinate to a global coordinate as a function of the third rotation matrix to yield a third global reaction force, and the fourth local reaction force from a local coordinate to a global coordinate as a function of the fourth rotation matrix to yield a fourth global reaction force, wherein the global reaction forces and moments for the sensor pad unit may be a summation of the first, second, third, and fourth global reaction forces. At least one of the first, second, third, and fourth calibration coefficients may be determined experimentally. At least one of the plurality of load cells may include twelve foil strain gages. Each of the plurality of load cells may include three full Wheatstone strain gage bridges.

The disclosure comprises the following clauses:

1. A dynamic load sensor system for use in microgravity environments, the dynamic load sensor system comprising:

a first sensor pad unit and a second sensor pad unit, wherein each of the first sensor pad unit and the second sensor pad unit comprises a sensor base plate, a top plate, and a plurality of load cells positioned between the sensor base plate and the top plate;

a processing unit having a processor operatively coupled with an internal memory device; and

an operator interface module having a display device and a plurality of operator input devices.

2. The dynamic load sensor system of clause 1, wherein each of the plurality of load cells comprises twelve foil strain gages.

3. The dynamic load sensor system of clause 1 or 2, wherein each of the plurality of load cells comprises three full Wheatstone strain gage bridges.

4. The dynamic load sensor system of any of clauses 1 to 3, wherein the operator interface module enables an operator to navigate between a plurality of operational modes using one or more of the plurality of operator input devices.

5. The dynamic load sensor system of clause 4, wherein the plurality of operational modes includes a standby mode, one or more work out modes, a download mode, and an upload mode.

6. The dynamic load sensor system of clause 5, wherein the one or more work out modes includes a set up mode and a run mode.

7. The dynamic load sensor system of any preceding clause, wherein each of the first sensor pad unit and the second sensor pad unit are configured to non-invasively couple with a preexisting exercise device.

8. The dynamic load sensor system of clause 7, wherein the preexisting exercise device is a resistive exercise device.

9. The dynamic load sensor system of any preceding clause, wherein each of the processing unit, the operator interface module, the first sensor pad unit, and the second sensor pad unit are separate components, and the processing unit is operatively coupled with the operator interface module, the first sensor pad unit, and the second sensor pad unit via a plurality of interconnecting cables.

10. The dynamic load sensor system of any preceding clause, wherein at least one of the processing unit or the operator interface module includes a data port communicatively coupled with the processor, the data port being configured to removably couple with an external memory device.

11. The dynamic load sensor system of any preceding clause, wherein the top plate comprises a first surface residing in a first plane and a second surface residing in a second plane that is substantially parallel to the first plane, wherein the first surface is shaped to include a geometric pattern.

12. The dynamic load sensor system of clause 11, wherein the geometric pattern is a triangular pattern.

13. The dynamic load sensor system of clause 11, wherein the first surface includes a network of ribs and spars to reinforce a least a portion of a perimeter of the top plate.

14. The dynamic load sensor system of clause 12, wherein the second surface includes a gripping material.

15. The dynamic load sensor system of clause 12, wherein the second surface includes a plurality of bench leg holes/slots to secure an exercise bench.

16. The dynamic load sensor system of any preceding clause, wherein the sensor base plate comprises a first surface residing in a first plane and a second surface residing in a second plane that is substantially parallel to the first plane, wherein the first surface includes a plurality of sensor recesses to secure the plurality of load cells.

17. The dynamic load sensor system of clause 16, wherein the first surface further comprises a hard-stop rim along a least a portion of a perimeter of the sensor base plate to prohibit the top plate from compressing in the event of overloading via the top plate, thereby mitigating damage to the load cells.

18. A method for performing load sensor calibration of a sensor pad unit in a dynamic load sensor system, the sensor pad unit having a plurality of load cells positioned between a sensor base plate and a top plate, the method comprising:

determining a first calibration coefficient for a first load cell having a first rotation angle, and a second calibration coefficient for a second load cell having a second rotation angle;

converting a first local load cell voltage from the first load cell to a first local reaction force using the first calibration coefficient, and a second local load cell voltage from the second load cell to a second local reaction force using the second calibration coefficient;

determining a first rotation matrix for the first load cell as a function of the first rotation angle, and a second rotation matrix for the second load cell as a function of the second rotation angle;

transforming the first local reaction force from a local coordinate to a global coordinate as a function of the first rotation matrix to yield a first global reaction force, and the second local reaction force from a local coordinate to a global coordinate as a function of the second rotation matrix to yield a second global reaction force;

determining a summation matrix to sum the first global reaction force and the second global reaction force; and

summing the first global reaction force and the second global reaction force using the summation matrix to provide global reaction forces and moments for the sensor pad unit.

19. The method of clause 18, further comprising the steps of:

determining a third calibration coefficient for a third load cell having a third rotation angle, and a fourth calibration coefficient for a fourth load cell having a fourth rotation angle;

converting a third local load cell voltage from the third load cell to a third local reaction force using the third calibration coefficient, and a fourth local load cell voltage from the fourth load cell to a fourth local reaction force using the fourth calibration coefficient;

determining a third rotation matrix for the third load cell as a function of the third rotation angle, and a fourth rotation matrix for the fourth load cell as a function of the fourth rotation angle; and

transforming the third local reaction force from a local coordinate to a global coordinate as a function of the third rotation matrix to yield a third global reaction force, and the fourth local reaction force from a local coordinate to a global coordinate as a function of the fourth rotation matrix to yield a fourth global reaction force,

wherein the global reaction forces and moments for the sensor pad unit is a summation of the first, second, third, and fourth global reaction forces.

20. The method of clause 19, wherein each of the first, second, third, and fourth calibration coefficients is determined experimentally.

21. The method of clause 20, wherein each of the plurality of load cells comprises twelve foil strain gages.

22. The method of clause 20, wherein each of the plurality of load cells comprises three full Wheatstone strain gage bridges.

DESCRIPTION OF THE DRAWINGS

These and other advantages of the present disclosure may be readily understood with the reference to the following specifications and attached drawings wherein:

Figures 1a and 1b illustrate an example Advanced Resistive Exercise Device (ARED).

Figures 2a through 2c illustrate an example dynamic load sensor system.

Figure 3 illustrates exemplary mounting locations of the load cells.

Figure 4 illustrates a top plan view of an inner surface of an exemplary sensor base plate.

Figures 5a and 5b illustrate top plan views of the inner and the outer surfaces of an exemplary top plate.

Figures 6a through 6c illustrate example sensor pad units installed on the ARED.

Figure 7 illustrates an exemplary process diagram for the various operational modes.

Figure 8 illustrates an exemplary calibration fixture.

Figure 9 illustrates a planar view of a dimensioned sensor pad unit with five different exemplary fixture mounting locations.

Figures 10a and 10b illustrate exemplary orientations of the sensor pad unit during a sensor pad unit calibration procedure.

Figure 11 illustrates exemplary coordinate systems and load cell positioning variables.

Figures 12a through 12d illustrate exemplary orientations of the load cells during a load cell calibration procedure.

Figures 13a and 13b illustrate exemplary squat and deadlight sensor unit data during parabolic flight testing.

Figures 14a through 14c illustrate exemplary sensor unit data during parabolic flight testing.

Figures 15a and 15b illustrate exemplary static calibration verification graphs in 1g and 0g.

## DETAILED DESCRIPTION

Preferred embodiments of the present disclosure may be described hereinbelow with reference to the accompanying drawings. In the following description, well-known functions or constructions are not described in detail because they may obscure the disclosure in unnecessary detail. For this disclosure, the following terms and definitions shall apply.

The terms "circuits" and "circuitry" refer to physical electronic components (*i.e.,* hardware) and any software and/or firmware ("code") which may configure the hardware, be executed by the hardware, and or otherwise be associated with the hardware. As used herein, for example, a particular processor and memory may comprise a first "circuit" when executing a first set of one or more lines of code and may comprise a second "circuit" when executing a second set of one or more lines of code.

The term "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "*e.g.,*" and "for example" set off lists of one or more non-limiting examples, instances, or illustrations.

The terms "about" and "approximately," when used to modify or describe a value (or range of values), mean reasonably close to that value or range of values. Thus, the embodiments described herein are not limited to only the recited values and ranges of values, but rather should include reasonably workable deviations. As utilized herein, circuitry or a device is "operable" to perform a function whenever the circuitry or device comprises the necessary hardware and code (if any is necessary) to perform the function, regardless of whether performance of the function is disabled, or not enabled (*e.g.,* by a user-configurable setting, factory trim, etc.).

The term "aircraft" refers to a machine capable of flight, including, but not limited to, both traditional runway and vertical takeoff and landing ("VTOL") aircraft. VTOL aircraft may include fixed-wing aircraft (*e.g.,* Harrier jets), rotorcraft (*e.g.,* helicopters), and/or tilt-rotor/tilt-wing aircraft.

The terms "communicate" and "communicating" refer to (1) transmitting, or otherwise conveying, data from a source to a destination, and/or (2) delivering data to a communications medium, system, channel, network, device, wire, cable, fiber, circuit, and/or link to be conveyed to a destination. The term "database" as used herein means an organized body of related data, regardless of the manner in which the data or the organized body thereof is represented. For example, the organized body of related data may be in the form of one or more of a table, a map, a grid, a packet, a datagram, a frame, a file, an e-mail, a message, a document, a report, a list, or data presented in any other form.

The terms "coupled," "coupled to," and "coupled with" as used herein, each mean a relationship between or among two or more devices, apparatuses, files, circuits, elements, functions, operations, processes, programs, media, components, networks, systems, subsystems, and/or means, constituting any one or more of: (i) a connection, whether direct or through one or more other devices, apparatuses, files, circuits, elements, functions, operations, processes, programs, media, components, networks, systems, subsystems, or means; (ii) a communications relationship, whether direct or through one or more other devices, apparatuses, files, circuits, elements, functions, operations, processes, programs, media, components, networks, systems, subsystems, or means; and/or (iii) a functional relationship in which the operation of any one or more devices, apparatuses, files, circuits, elements, functions, operations, processes, programs, media, components, networks, systems, subsystems, or means depends, in whole or in part, on the operation of any one or more others thereof.

The term "data" as used herein means any indicia, signals, marks, symbols, domains, symbol sets, representations, and any other physical form or forms representing information, whether permanent or temporary, whether visible, audible, acoustic, electric, magnetic, electro-magnetic, or otherwise manifested. The term "data" is used to represent predetermined information in one physical form, encompassing any and all representations of corresponding information in a different physical form or forms.

The term "database" as used herein means an organized body of related data, regardless of the manner in which the data or the organized body thereof is represented. For example, the organized body of related data may be in the form of one or more of a table, map, grid, packet, datagram, frame, file, email, message, document, report, list, or in any other form.

The term "exemplary" means "serving as an example, instance, or illustration." The embodiments described herein are not limiting, but rather are exemplary only. It should be understood that the described embodiments are not necessarily to be construed as preferred or advantageous over other embodiments. Moreover, the terms "embodiments of the invention," "embodiments," or "invention" do not require that all embodiments of the invention include the discussed feature, advantage, or mode of operation.

The term "memory device" means computer hardware or circuitry to store information for use by a processor. The memory device can be any suitable type of computer memory or any other type of electronic storage medium, such as, for example, read-only memory (ROM), random access memory (RAM), cache memory, compact disc read-only memory (CDROM), electro-optical memory, magneto-optical memory, programmable read-only memory (PROM), erasable programmable read-only memory (EPROM), electrically-erasable programmable read-only memory (EEPROM), a computer-readable medium, or the like.

The term "processor" means processing devices, apparatuses, programs, circuits, components, systems, and subsystems, whether implemented in hardware, tangibly embodied software, or both, and whether or not it is programmable. The term "processor" includes, but is not limited to, one or more computing devices, hardwired circuits, signal-modifying devices and systems, devices and machines for controlling systems, central processing units, programmable devices and systems, field-programmable gate arrays, application-specific integrated circuits, systems on a chip, systems comprising discrete elements and/or circuits, state machines, virtual machines, data processors, processing facilities, and combinations of any of the foregoing. The processor may be, for example, any type of general purpose microprocessor or microcontroller, a digital signal processing (DSP) processor, an application-specific integrated circuit (ASIC). The processor may be coupled to, or integrated with, a memory device.

Disclosed herein is a self-contained enhanced dynamic load sensing system. As will be explained, the enhanced dynamic load sensing system can collect biomechanical force and moment data in a microgravity environment. For example, the enhanced dynamic load sensing system may be integrated (*e.g.*, removably coupled) with another device, such as the ARED. As it relates to the ARED, for example, the disclosed enhanced dynamic load sensing system introduces improvements to the existing 6-DOF force and moment sensors, while decreasing the overall height of the sensors, separating the electronics into an off-board base unit, providing footprint size and dimensions to cover the surface of the ARED platform, providing a real-time operator interface module, and other improvements. The enhanced dynamic load sensing system is advantageous in that it provides both immediate force feedback to exercising crewmembers aboard the International Space Station using the ARED and collect forces and moment data for analysis by researchers on the ground (*e.g.,* at a mission control center).

A dynamic load sensor system, as disclosed herein, offers a number of advantageous features and performance parameters. First, the dynamic load sensor system 200 can measure forces and moments from three orthogonal axes, while complying with a predetermined sensor pad unit footprint. That is, the dynamic load sensor system provides increased load capacity and load measurement accuracy over a larger footprint. Second, the dynamic load sensor system provides sensor pad units with an overall force capacity of 400 pound-force (lbf) and a 600 lbf capacity within the load cell box defined by the four load cells 248. Third, the dynamic load sensor system demonstrates compliance with force and moment error requirements and provides a data sampling rate in the range of 250-1000 Hz. Fourth, the dynamic load sensor system 200 incorporates an operator interface module to provide real-time (or near real-time) feedback to the operator (*e.g.*, the crewmember). Finally, the dynamic load sensor system demonstrate compliance with mitigation of load inaccuracies due to platform and device bending and torsion, while also supporting continuous long-term use of not less than 6 months.

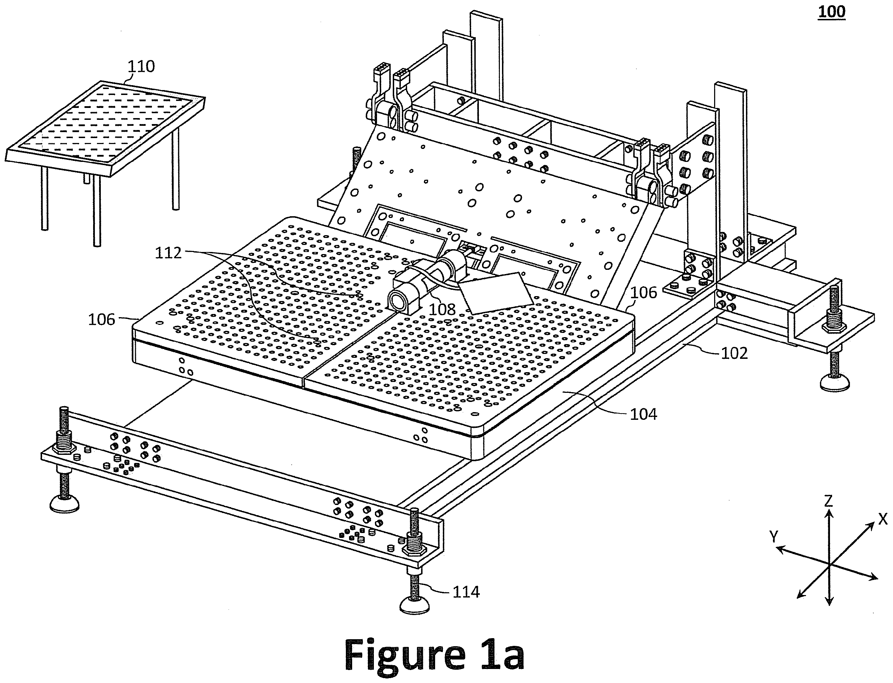

Figure 1a illustrates an example advanced resistive exercise device (ARED) 100, which functions to maintain crew health in microgravity environments (*e.g.*, aboard spacecraft, such as the International Space Station). For example, crewmembers aboard the International Space Station are encouraged to exercise daily on the ARED 100 to maintain their preflight muscle, bone strength, and endurance. Indeed, extravehicular activity (EVA), intravehicular activity (IVA), re-entry, and emergency egress activities necessitate the crewmembers' continued strength and endurance. The ARED 100 offers the ability to exercise all major muscle groups, while focusing on the primary resistive exercise routines: squats; dead lifts; and calf raises. The ARED 100 is designed to accommodate all crewmember sizes and provides a load of up to 600 pounds, along with a display that makes it easier for crewmembers to follow a personalized exercise plan. As illustrated, the ARED 100 includes a number of assemblies, including a structural frame 102, an exercise platform subassembly 104, a pulley assembly 108, and an exercise bench 110. The structural frame 102 may be bolted directly to a surface of the spacecraft, or via a plurality of adjustable feet 114 (*i.e.,* adjustable in length along the z-axis using, for example, threaded rods) to enable adjustment of the structural frame 102 relative to the surface of the spacecraft.

The exercise platform subassembly 104 mounts to the structural frame 102 to provide the surface from which the crewmember may perform their exercise routines. The platform subassembly 104 includes two force plates 106 that act as the reaction surface during the exercise routines. A plurality of load cells (*e.g.,* 3 to 6, more preferably 4) is installed beneath each force plate 106 to measure the reactive loads imparted by the crewmember. A mechanical pulley assembly 108 may be provided at the front end of the platform subassembly 104 (between the two force plates 106) to enable crewmembers to perform cable-based exercise routines. The pulley assembly 108, for example, can interface an exercise rope to an arm base assembly to provide load for the exercise routines. To facilitate shoulder presses, bench presses, and other seated or lying exercise routines, an exercise bench 110 may be removably mounted to the platform subassembly 104 (*e.g.,* atop the force plates 106). For example, the legs of the exercise bench 110 may be sized and shaped to be inserted and locked into a plurality of bench interface mechanisms 112. In one aspect, the exercise bench 110 includes four legs to couple with four bench interface mechanisms 112, where two bench interface mechanisms 112 are positioned on each force plate 106. The exercise bench 110 may be folded and stowed when not in use.

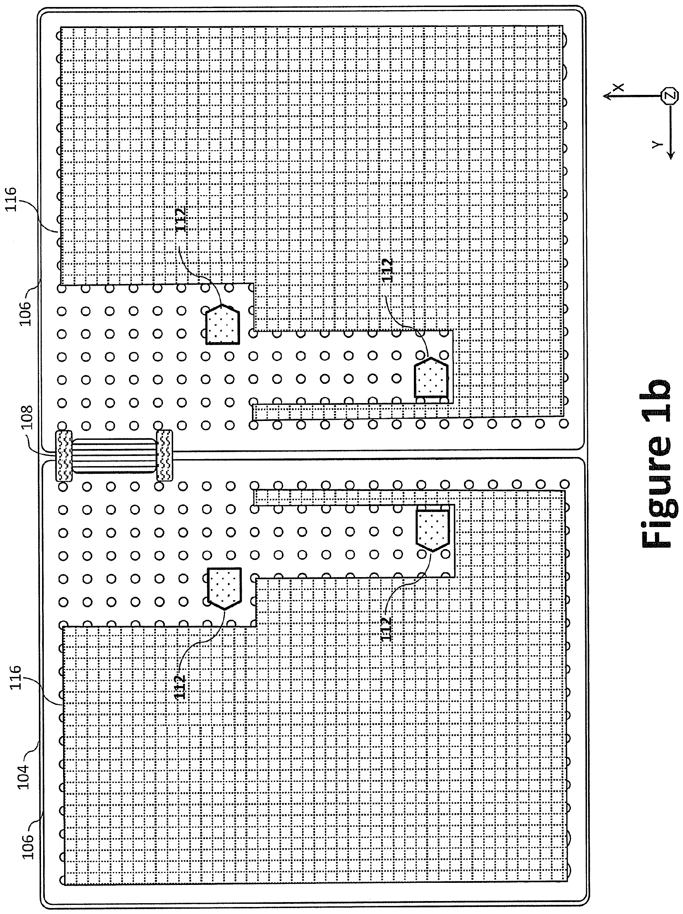

Figure 1b illustrates a top plan view of the exercise surface of the ARED's 100 platform subassembly 104 (*i.e.,* the force plates 106). To reduce the risk of slipping, a gripping material 116 (*e.g.,* slip-resistant tape, or sheet material) may be provided on the top surface of each of the force plates 106 to increase the friction between the force plates 106 and the crewmember's shoes. The gripping material 116 may be sized and shaped to accommodate the pulley assembly 108 and the bench interface mechanism 112.

While omitted from the Figures 1a and 1b, the ARED may also include a number of other components, such as a cylinder / flywheel assembly, a main arm assembly, an arm base assembly, and a heel block assembly. The cylinder / flywheel assembly may be used to provide a constant resistive force for the various exercise routines. The mechanical vacuum canisters provide the primary force, while the flywheels are used to simulate the inertial component of the exercise as would be experienced on the ground. The resistive load can be adjusted by turning a load adjustment handle to move the attachment point of the piston rods, thereby changing the length of the lever arm. A main arm assembly includes the wishbone arm and the lift bar components. Load cells can be installed in the lift bar struts and the associated cables have been attached. Arm base assembly includes the load adjustment mechanism, interfaces for the cylinder / flywheel assembly, main arm assembly, cable pulley assembly, and the platform subassembly 104. Two load cells, one rotational sensor, and associated cables may be installed to measure the reactive loads during cable-based exercise routines. Finally, a heel block assembly may mount to the platform subassembly 104 as an accessory to allow the crewmember to perform heel-raise exercise routines. Like the exercise bench 110, the heel block assembly can be removed, folded, and stowed when not in use. The ARED 100 may further include an instrumentation system having triaxial force sensors located in the platform subassembly 104 that are configured to record force in three dimensions. In addition, load sensors in the main lift arm and the arm base assembly measure unidirectional forces. The arm base assembly also has rotational sensors that record the range of motion of the arm.

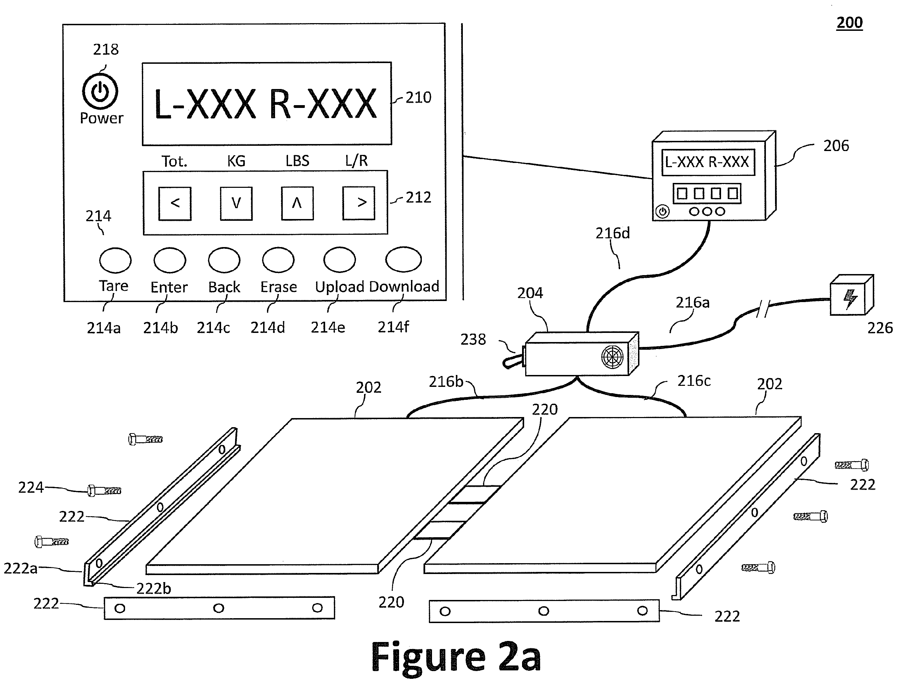

Figures 2a through 2c illustrate an example dynamic load sensor system 200 to measure the kinetics of crewmembers in microgravity (*e.g.*, aboard a spacecraft). Specifically, the illustrated dynamic load sensor system 200 is configured for use aboard the International Space Station as crewmembers exercise using the ARED 100. The dynamic load sensor system 200 generally comprises two sensor pad units 202, a processing unit 204, and an operator interface module 206, as well as various interconnecting cables 216 and mounting hardware (*e.g.*, interface brackets 222 and fasteners 224).

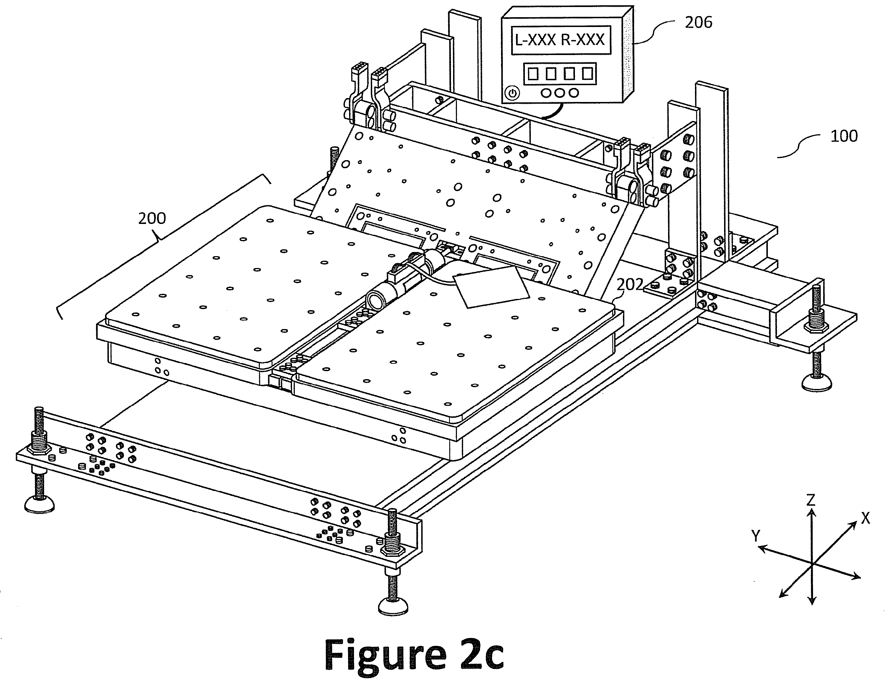

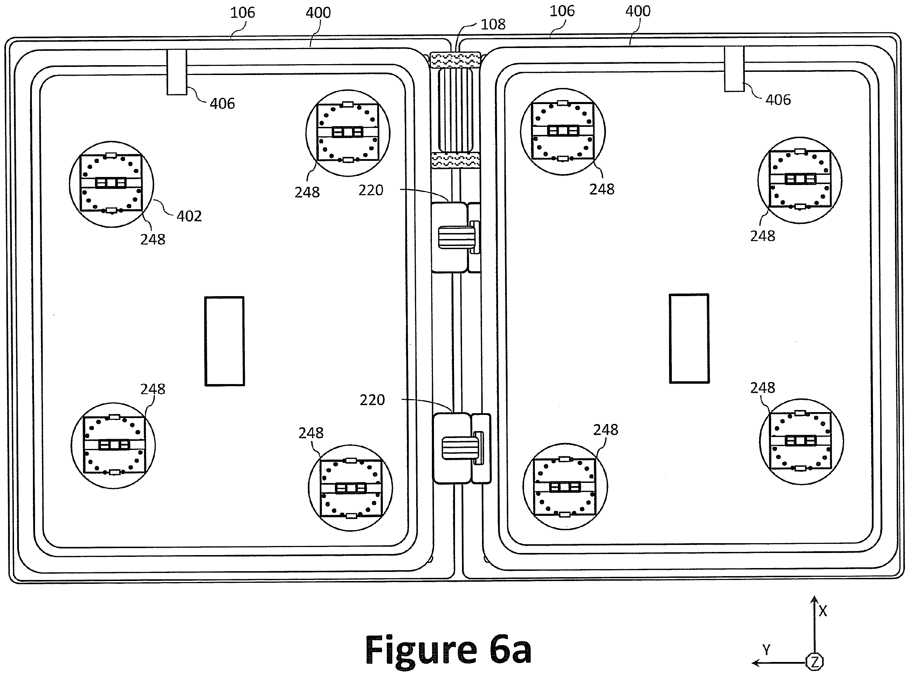

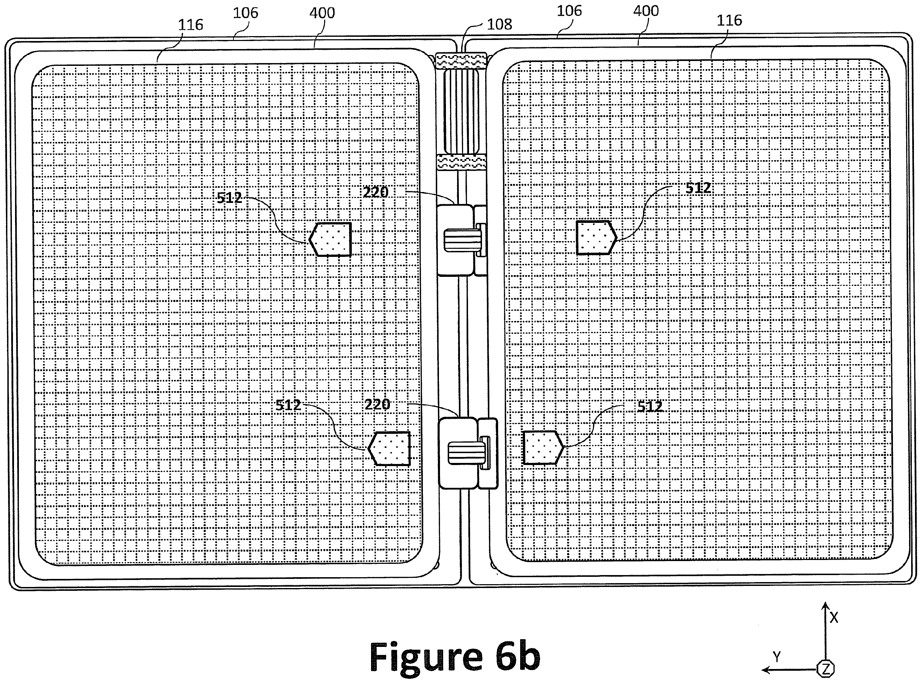

The illustrated sensor pad units 202 are sized and shaped for installation on the force plates 106 of the ARED 100 (as best illustrated in Figures 2c, 6a, and 6b). The dynamic load sensor system 200 benefits from a unique, non-permanent and non-invasive technique for installing the two sensor pad units 202 on the ARED 100 (or another preexisting exercise device) under microgravity conditions without requiring modification of the force plates 106. The adaptation included: design of interface brackets 222 (*e.g.,* linear L-shaped brackets) that attach to the sensor pad units 202 to enable its installation on the force plates 106. Further, the two sensor pad units 202 may be removably coupled to one another using a set of engagement latches 220 (*e.g.,* spring latches). For example, the incorporation of engagement latches 220 provides the required retention forces and facilitates an installation/removal protocol for use under microgravity conditions.

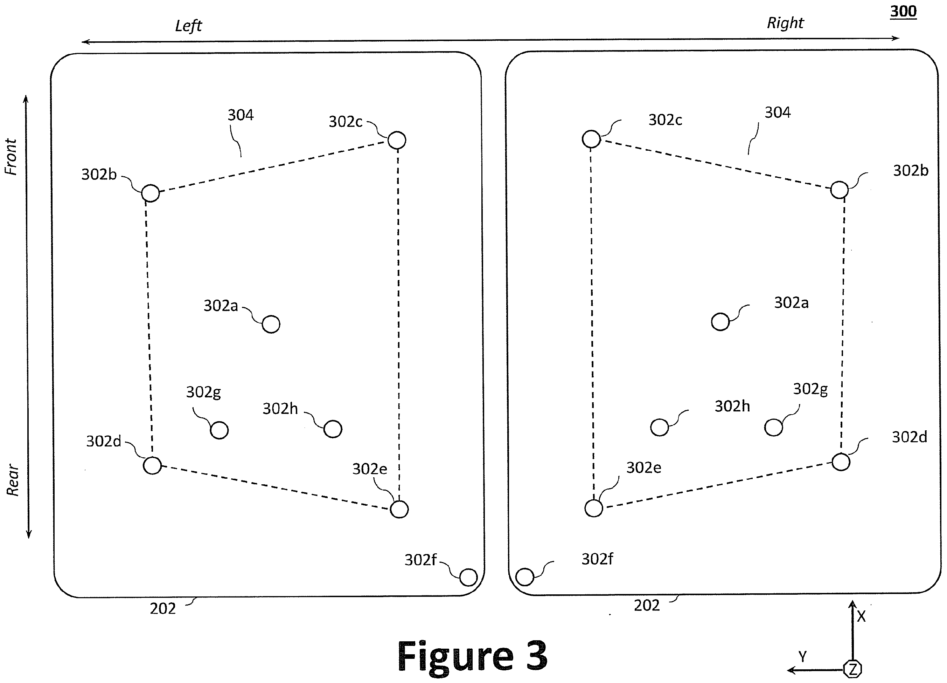

Each sensor pad unit 202 may be configured to measure crew-induced loads imparted to the platform subassembly 104. The overall form factor of the sensor pad units 202 is designed to minimize disruption to a crewmember's exercise form, thereby mitigating impact on the crewmember that could result from implementation of the dynamic load sensor system 200 on the ARED 100. For example, each sensor pad unit 202 may provide a footprint that is 12 to 24 inches wide (W) by 18 to 32 inches in height (H), more preferably about 19 inches (W) x 24 inches (H). Each sensor pad unit 202 employs a plurality (*e.g.,* two to six, more preferably, four) of instrumented load cells 248 to measure 3-axis forces and 3-axis moments to providing 6 degrees of freedom at a 250 Hz rate. Each load cell 248 may employ, for example, three full Wheatstone strain gage bridges (*i.e.,* four foil strain gages per bridge). Full scale load measurement by each sensor pad unit 202 may provide up to 400 lbf (1779 N) overall, or about 600 lbf (2669 N) with-in the load cell box defined by the four (or more) load cells 248 (see Figure 3). As will be discussed, each sensor pad unit 202 generally comprises a sensor base plate 400 and a top plate 500, where the load cells 248 are positioned (*e.g.,* sandwiched) in a hollow cavity therebetween.

The processing unit 204 may include one or more processors 228, a clock 230, an internal memory device 232, a data port 234, power management circuitry 236, a physical power switch 238, and a communication module 246. The clock 230 is coupled to the one or more processors 228 to provide clock, timing signals, and/or pulses thereto. The processing unit 204 is provided and designed for installation adjacent (and/or behind) the ARED 100. The processing unit 204 facilitates, *inter alia,* the collection of data from each of the sensor pad units 202, sensor pad unit 202 signal conditioning/filtering, onboard storage of sensor data (*e.g.,* via the internal memory device 232), data transfer to an external memory device 240 (*e.g.,* via data port 234), power conditioning and distribution via the power management circuitry 236, and overall control of the dynamic load sensor system 200 via the one or more processors 228.

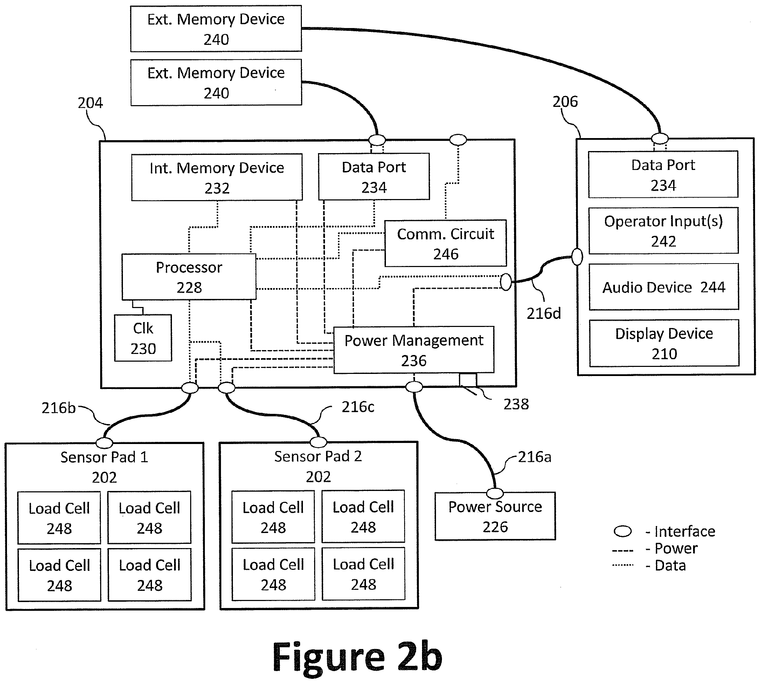

The one or more processors 228 may be communicatively coupled with the various components of the 200, including the internal memory device 232, the external memory device(s) 240 (via one or both data ports 234), the communication module 246, the two sensor pad units 202, and the operator interface module 206. The internal memory device 232 may include read-only memory (ROM) to receive one or more instruction sets, random access memory (RAM) having a plurality of buffers for temporarily storing and retrieving information, and an internal data storage device, such as a hard drive, a solid state drive or other non-volatile data storage device. The external memory device 240 may be, for example, a flash memory drive or other memory device. The data port 234 may be, for example, a universal serial bus (USB) port (*e.g.,* complying with USB specifications 1.0 to 2.0 or 3.0 to 3.1, with connector types A, B, C, mini-A, mini-B, micro-A, micro-B, etc.). One or more circuits may be provided between the sensor pad units 202 and the processor 228 to condition and/or filter the signals from the sensor pad units 202. Therefore, while the processor 228 is illustrated in Figure 2b as a single component coupled directly to various other components, one of skill in the art would recognize that additional processors and/or circuits may be provided (or incorporated into the one or more processors 228) to process power and/or signals.

The power management circuitry 236 provides power conditioning and power distribution to the various components of the dynamic load sensor system 200, including the sensor pad units 202 and the operator interface module 206. The components of the processing unit 204 may operate using a DC power supply (*e.g.,* a 28 volt DC power supply). To accommodate various forms of power sources 226 (*e.g.,* 120VAC line current, the International Space Station's electrical system, which operates at 120 to 160 volts DC), the power management circuitry 236 may include one or more AC-DC converters and/or DC/DC converters, such as step-down, buck-boost, boost, buck, or flyback converters. The power management circuitry 236 may also include one or more filters (*e.g.,* electromagnetic filters) to mitigate interference from other devices, such as electromagnetic interference (EMI). In certain aspects, the power management circuitry 236 may include, or be operatively coupled to, a backup battery. The power supply to the power management circuitry 236 may be toggled on and off via a physical power switch 238.

The communication module 246 includes circuitry to manage communication and/or transmission of signals or data between the dynamic load sensor system 200 (*e.g.,* the processing unit 204) and another device, such as the ARED 100, the spacecraft, a portable user device (*e.g.,* a watch, a phone, a tablet computer, laptop, etc.), etc. For example, the communication module 246 may communicate status updates (*e.g.,* regarding the crewmember/exercise routine) from the processing unit 204 to another device to facilitate remote monitoring of the crewmember or the dynamic load sensor system 200. The communication module 246 may also be used to provide maintenance to the dynamic load sensor system 200. For example, the communication module 246 may be used to perform diagnostics, to communicate software updates to the processing unit 204, etc.

In certain aspects, one or more vital sensors may be positioned on the crewmember to monitor one or more physiological states of the crewmember before, during, and/or after an exercise routine. The vital sensors may include, *inter alia,* heart rate monitors, blood pressure sensors 606b, and other vital monitors, such as glucose monitoring; cardiovascular monitoring and event recording; neurological monitoring (*e.g.,* electroencephalogram (EEG)) tests; and sleep monitoring devices. The one or more physiological states may include cardiac parameters (*e.g.,* ballistocardiograms, heart rhythm, heart rate in beats per minute (bpm), etc.), hemodynamic parameters (*e.g.,* blood pressure and blood flow), and other vitals. The vital sensors may be embodied as wearable sensors to be worn by the crewmember, each having a unique identification number that can be correlated with a specific crewmember. For example, wearable vital sensors may be provided as a wrist band, chest band, head bands, or other devices, such as garments, hats, socks, shoes, eyeglasses, wristwatches, headphones, and even portable user devices (*e.g.,* the crewmember's portable computer, portable digital assistant (PDA), smart phone, etc.). In another aspect, the one or more fiducial markers may be positioned on the crewmember to monitor his or her movement. For example, one or more cameras may image/monitor fiducial markers to track movement, gait analysis for identifying any abnormalities in the crewmember's movement. The fiducial markers can be unique to each crewmember and/or can be machine readable for associating member to crewmember ID.

The communication module 246 may employ wired (*e.g.,* using the Ethernet) or wireless transceivers. For example, a wireless transceiver (and antenna) may communicate via one or more wireless standards such as Bluetooth (*e.g.,* short-wavelength, ultra-high frequency (UHF) radio waves in the industrial, scientific, and medical (ISM) band from 2.4 to 2.485 GHz), near-field communication (NFC), Wi-Fi (*e.g.,* Institute of Electrical and Electronics Engineers' (IEEE) 802.11 standards), etc. By way of illustration, to avoid entanglement, the communication module 246 may wirelessly communicate with the vital sensors/wearable sensors to track the crewmember's physiological state.

The dynamic load sensor system 200 further includes an operator interface module 206 to enable display, *inter*

*alia,* feedback, progress, exercise prescriptions, etc. The exercise prescriptions (*i.e.,* proscribed exercise routines) may be sent from the ground (*e.g.,* a mission control center) to the dynamic load sensor system 200. The operator interface module 206 is designed for mounting adjacent to the ARED 100 and within the crewmember's field-of-view (and field-of-reach). The operator interface module 206 functions as the primary user interface for operational control of the dynamic load sensor system 200. The operator interface module 206 may include a display device 210, a data port 234, an audio device 244 (*e.g.,* speaker, microphone, etc.), and a plurality of operator input devices 242 (*e.g.,* selector buttons 212, function buttons 214, a power button 218, etc.). The display device 142 may include one or more light emitting diodes (LEDs), a liquid crystal display (LCD) screen, a segmented display device, etc. For example, the display device 142 may be an alphanumeric segmented LED/LCD display or a matrix LCD display. In certain aspects, the operator input devices 242 may be provided as soft buttons via a graphical user interface (GUI) of the display device 210, where an input from the crewmember is detected (received) via a touch screen (*e.g.,* a thin layer of sensing circuitry overlaying the display device 210).

In operation, the operator interface module 206 provides real-time digital visual feedback of the forces imparted by a crewmember upon the sensor pad units 202 (*e.g.,* while they use the ARED 100). Therefore, the operator interface module 206 provides real-time loading information to the crewmember and a definition of crew interactions with the system. The plurality of selector buttons 212 may include, *inter alia,* up, down, left, and right arrow buttons, while the plurality of function buttons 214 may include, for example, a tare button 214a, an enter button 214b, a back button 214c, an erase button 214d, a data upload button 214e, and a data download button 214f. An exercise prescription can be automatically loaded into an individual crewmember profile, which can be accessed during an exercise session. During exercise, the load and number of repetitions will be simultaneously recorded and displayed on the display device 210 of the operator interface module 206. The recorded data can also be automatically and/or periodically downloaded to an on-board server via the communication module 246, which may then be communicated to the ground. The power button 218 may be used to initiate a power down procedure (*e.g.,* a shutdown mode 716) before cutting power to the processing unit 204 via the physical power switch 238. In certain aspects, one or more cameras (whether still cameras or video cameras) may be positioned adjacent the 200 to monitor the crewmember during an exercise routine to ensure that the exercises are being properly performed (*e.g.,* to monitor the crewmember's form). The camera feed from the one or more cameras (which may be associated with the crewmember's identification number (ID)) may be stored to the internal memory device 232 or communicated to another device via the communication module 246.

The various components of the dynamic load sensor system 200 may be interconnected via a plurality of interfaces and interconnecting cables 216. The interfaces may be hardwired or removably coupled (*e.g.,* using a plug and socket) with one another. For example, interconnecting cables 216 may be used to interconnect the various hardware components of the dynamic load sensor system 200, while allowing for flexibility in their placement and mounting. As illustrated, the interconnecting cables 216 may include a rack power cable 216a for supplying electrical power from a power source 226 (*e.g.,* the International Space Station) to the processing unit 204, two sensor cable assemblies 216b, 216c to couple the sensor pad units 202 to the processing unit 204, and an interface cable assembly 216d to couple the operator interface module 206 with the processing unit 204. Each of the interconnecting cables 216 may be a multi-conductor cable with connectors (*e.g.,* plugs) at each end (*i.e.,* the distal and proximal ends) and configured to carry power and/or data signals.

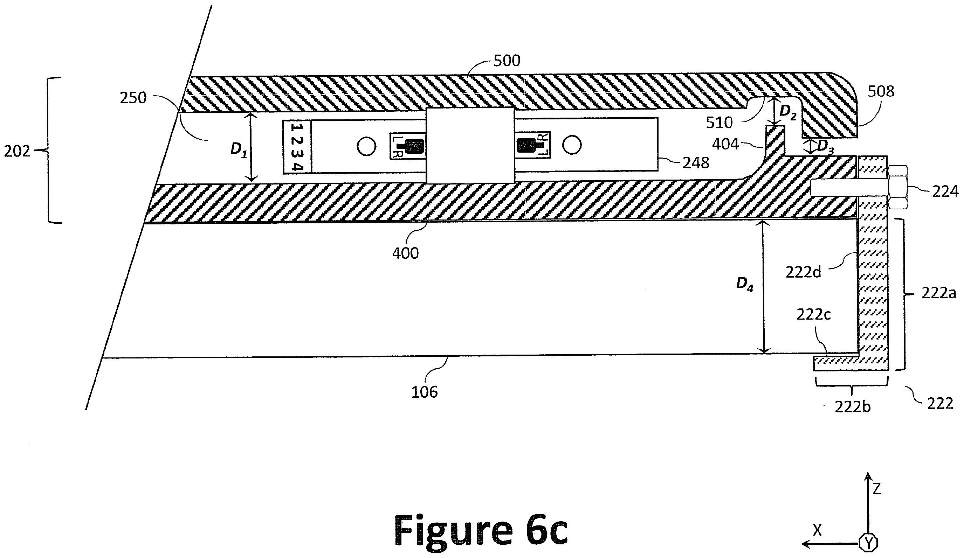

An advantage of the dynamic load sensor system 200 is that it can be quickly and noninvasively installed on (or removed from) the ARED 100 (or another device). To that end, the sensor pad units 202 may be non-invasively and/or removably coupled to the force plates 106 of the ARED 100 using non-invasive mounting hardware. The non-invasive mounting hardware may include, for example, a plurality of interface brackets 222 that can be fastened along the perimeter of the sensor pad units 202 using a plurality of fasteners 224 (*e.g.,* bolts, screws, pins, snaps, etc.). As illustrated, each of the interface brackets 222 includes an attachment portion 222a and a lip portion 222b, where the attachment portion 222a fastens to the sensor pad units 202 and the lip portion 222b is sized and shaped to slip under the edge of a force plate 106 to secure the force plate 106 against the sensor pad units 202 (as best illustrated in Figure 6c). Figure 2c illustrates a perspective view of dynamic load sensor system 200 noninvasively coupled with the ARED 100.

The location and orientation of the load cells 248 has been optimized to meet the desired load capacity, load accuracy, and measurement spatial footprint. Specifically, load cell position and orientation was optimized through detailed finite-element analysis of the structural elements and loading environment to provide an optimized load cell location and orientation. For example, Figure 3 illustrates a load cell layout map 300 for the left and right sensor pad units 202. The load cell layout map 300 illustrates a plurality of example load cell loading locations. Specifically, during testing, eight load cell loading locations were considered for each sensor pad unit 202 (*i.e.,* points 302a through 302h, which correspond to load cell loading locations A through H). In the case of the right sensor pad unit 202, load cell loading location F was mirrored to evaluate a worst case loading scenario on the inner rear corner for each sensor pad unit 202. During testing, load cell loading location F did not produce accurate results due to sensor saturation when the top plate 500 bottomed out on the sensor base plate 400, a consequence that may be addressed using the reinforcement using a network of ribs and spars and the hard-stop techniques discussed below. Locations G and H produced similar accuracy results to the load cell loading locations A through E. To provide a larger load cell box area 304 (as defined by the dotted line), Locations B, C, D, and E were selected for placement of the load cells 248. If five load cells 248 are desired, however, Location A may also be considered.

Each sensor pad unit 202 may be assembled using a sensor base plate 400 and a top plate 500, where the load cells 248 are positioned within a hollow cavity 250 defined between the sensor base plate 400 and the top plate 500. The sensor base plate 400, the top plate 500, and the load cells 248 may be fabricated from one or metals or metal alloys, including aluminum alloys, magnesium alloys, titanium alloys, and other lightweight, high strength alloys. In one example, the load cells 248 may be fabricated using an aluminum alloy, such as 7075 aluminum alloy, which contains zinc as the primary alloying element, although other alloys are contemplated. The sensor base plate 400, the top plate 500 may also be fabricated using an aluminum alloy, such as 6061 aluminum alloy, which contains magnesium and silicon as its major alloying elements. Each of the sensor base plate 400 and the top plate 500 comprises a first surface residing in a first plane and a second surface residing in a second plane that is substantially parallel to the first plane.

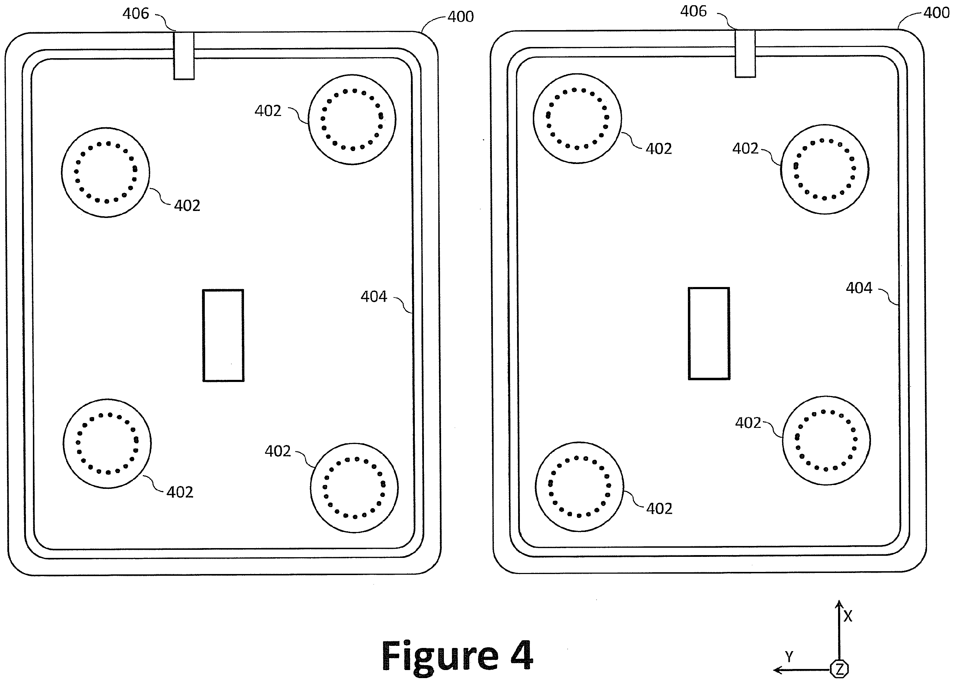

Figure 4 illustrates a top plan view of the inner surface (*i.e.*, the surface that faces the top plate 500) of an example sensor base plate 400. As illustrated, the inner surface of the sensor base plate 400 may be sized and shaped (*e.g.*, cast, milled and/or machined) to include a plurality of sensor recesses 402 (illustrated at locations B, C, D, and E), a hard-stop rim 404, and a cable passageway 406. The plurality of sensor recesses 402 are sized and shaped to receive/accommodate a desired load cell 248. The plurality of sensor recesses 402 may be circular to enable the load cell 248 to be positioned in any position (*e.g.*, rotated) relative to the sensor base plate 400. The hard-stop rim 404 may be positioned along the perimeter of the sensor base plate 400 to mitigate damage to the load cells 248 in the event of overloading. More specifically, when the sensor pad unit 202 is overloaded, the hard-stop rim 404 prohibits the top plate 500 from compressing (*i.e.*, traveling toward the sensor base plate 400 along the z-axis) beyond a predetermined point. The hard-stop rim 404 also assists in aligning the sensor base plate 400 vis-à-vis the top plate 500 by at least partially residing within the boundaries of an alignment lip 508 defined by the top plate 500. A cable passageway 406 may be provided at the front end of the sensor base plate 400 to enable the various cables and other conductors (*e.g.*, the interconnecting cables 216) to pass from the hollow cavity 250 of the sensor pad unit 202 to the exterior (*e.g.*, to couple with the processing unit 204) without the risk of damage that can result from being pinched between the sensor base plate 400 and the top plate 500.

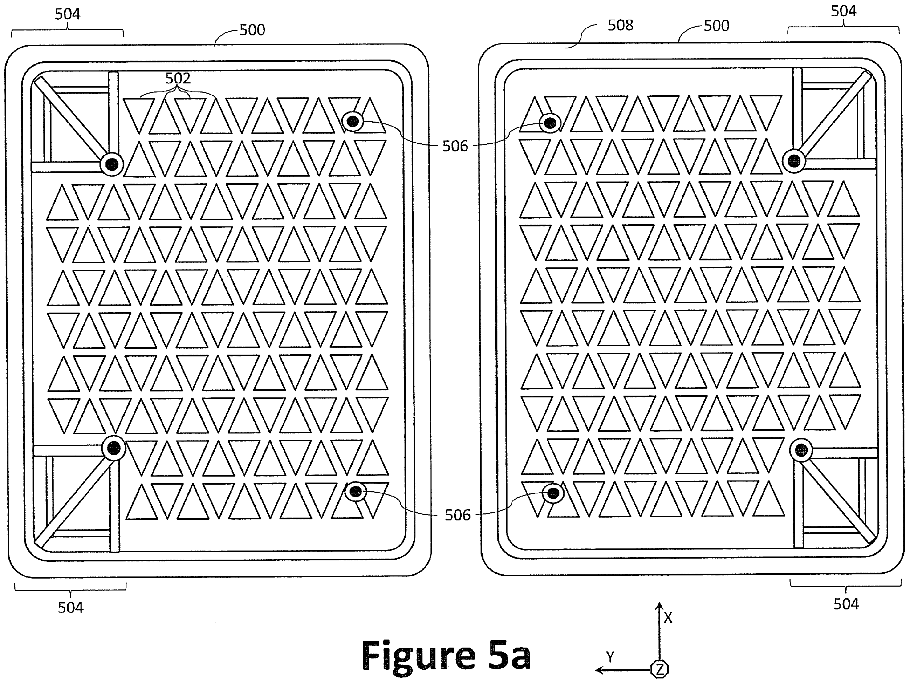

Figure 5a illustrates a top plan view of the inner surface (*i.e.*, the surface that faces the sensor base plate 400) of an example top plate 500. As illustrated, the inner surface of the top plate 500 may be configured (*e.g.*, cast, milled and/or machined) with a plurality of triangles 502 (collectively defining a triangular pattern) machined into the inner surface. The plurality of triangles 502 may be, for example, equilateral triangles, although isosceles triangles are contemplated. While a plurality of triangles 502 are illustrated, other geometric patterns and/or shapes are also possible, including quadrilaterals, pentagons, hexagons, heptagons, octagons, etc. For example, a plurality of hexagons may be provided to define a honeycomb pattern. An advantage of machining the inner surface (*e.g.*, with a triangular pattern) is that the weight of the top plate 500 can be reduced by about 40%, while maintaining rigidity. For example, a 19 (W) x 24 (H) top plate 500 with a triangular pattern milled into the inner surface weighs about 3.0 kg (6.5 lbs), while a comparable top plate without the milled triangular pattern weighs about 5.0 kg (11.0 lbs). Where weight is not a concern, however, the top plate 500 may be provided with a smooth inner surface. As best illustrated in Figure 6c, the inner surface of the top plate 500 includes an alignment lip 508 positioned along the perimeter of the top plate 500 to assist in aligning the top plate 500 vis-à-vis the sensor base plate 400 by retaining the hard-stop rim 404 within the boundaries of the alignment lip 508.

The inner surface of the outer corners (adjacent the lateral edges) of each top plate 500 may be provided with include a network of ribs and spars 504 to reduce plate flexing during corner and edge loading to improve sensor accuracy. The network of ribs and spars 504 may occupy any area that is, for example, 3 to 5 inches wide (W) by 4 to 6 inches in height (H), more preferably about 4 inches (W) x 5 inches (H). For example, the network of ribs and spars 504 may include three ribs (each about 0.2 to 0.6 inches thick, more preferably about 0.4 inches thick) located radially around the load cell mounting location (*e.g.*, locations B, C, D, and E) and connected via two cross members (each about 0.1 to 0.5 inches thick, more preferably about 0.3 inches thick). The network of ribs and spars 504 addresses a scenario where, for example, a crewmember is performing heel raises with only the front half of the foot on the top plate 500 at the corner of the top plate 500, with the heel extending beyond the rear edge. Testing results demonstrate that a top plate 500 with the network of ribs and spars 504 exhibits a 30% reduction in deflection during corner loading. In other aspects, a rib lattice structure may be provided to span the entire front and/or rear edges of each top plate 500, as well as the medial and/or lateral edges of each top plate 500. Such a continuous rib lattice structure would reinforce all areas where a crewmember could potentially stand during heel raises, sumo-, and single-legged squats to minimize bending. In addition, the stiffness of the top plate 500 (*e.g.*, but increasing thickness) may be increased to further reduce deflection and to improve sensor accuracy, thereby reducing the travel of the top plate 500 and minimizing the impact on the hard-stop rim 404.

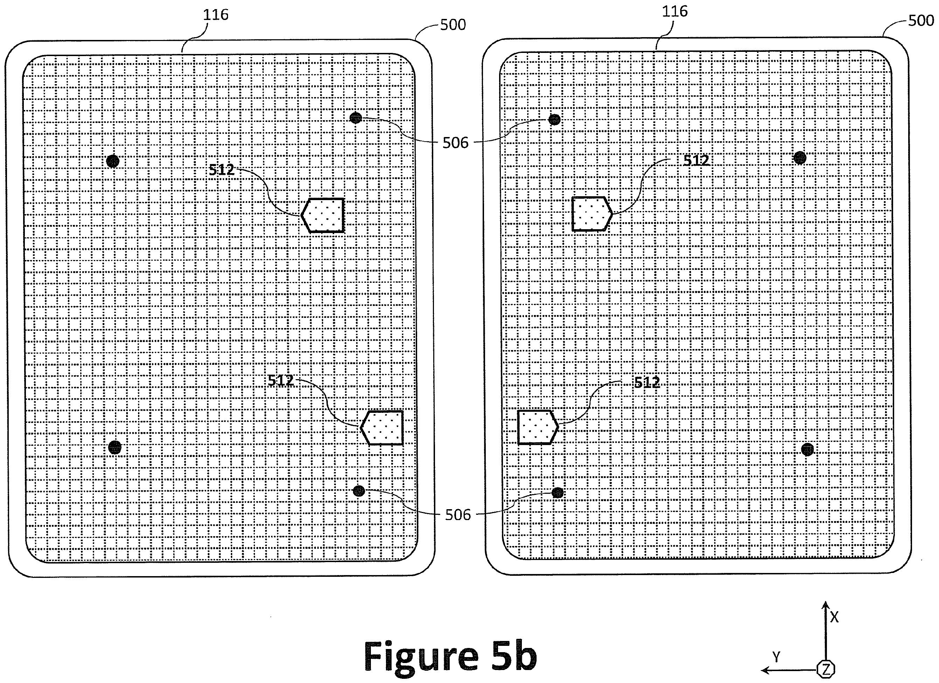

Figure 5b illustrates a top plan view of the outer surface (*i.e.*, the surface that faces away from the sensor base plate 400) of the example top plate 500. As illustrated, the outer surface of the top plate 500 may include a gripping material 116, a plurality of bench leg holes/slots 512, and a plurality of load cell access-holes 506. The top plate 500 may incorporate the bench leg holes/slots 512 (*e.g.,* cutouts) to provide access to the bench interface mechanism 112 of the platform subassembly 104, thereby obviating the need to remove the sensor pad units 202 from the platform subassembly 104 during each exercise session that involves the exercise bench 110. The leg holes/slots 512 should be sized to provide sufficient clearance to access the interface mechanism 112 that secures/locks the exercise bench 110 to the platform subassembly 104 (via the force plates 106). Further, the gripping material 116 serves to reduce the risk of slipping by increasing the friction between the top plates 500 and the crewmember's shoes. Each of the plurality of load cell access-holes 506 may be provided at a load cell mounting location to enable the operator to access the load cells 248 within the hollow cavity 250 without having to remove the top plate 500. For example, the operator may insert a tool through an access-hole 506 to adjust, rotate, or otherwise reposition a particular load cell 248.

Figures 6a and 6b illustrate top plan views of the sensor pad units 202 installed on the ARED 100, but in different stages of assembly. Specifically, Figure 6a illustrates a top plan view of the inner surface of the left and right sensor base plates 400 coupled to the platform subassembly 104 of the ARED 100 and to one another via a set of engagement latches 220. As illustrated, a load cell 248 is positioned within each sensor recess 402 of locations B, C, D, and E. Figure 6b illustrates a fully assembled set (*i.e.,* left and right) of sensor pad units 202 installed on the ARED 100.

Figure 6c illustrates a cross-sectional view of the lateral side of a sensor pad unit 202 coupled to the exercise platform subassembly 104 using non-invasive mounting hardware. As can be seen, cross sectional profiles of the top plate 500 and the sensor base plate 400 are shaped such that they define the hollow cavity 250 to house the plurality of load cells 248. As illustrated, the load cells 248 are positioned on the sensor base plate 400 and configured to support the weight of the top plate 500. The top plate 500 may be secured to each of the load cells 248 via a plurality of screws. As noted above, the hard-stop rim 404 positioned along the perimeter of the sensor base plate 400 mitigates damage to the load cells 248 in the event of overloading. Specifically, when overloaded, the hard-stop rim 404 contacts the valley of the hard-stop recess 510 of the top plate 500, thereby preventing further movement/compression. The hard-stop recess 510 is adjacent, and partially defined by, the alignment lip 508. By way of illustration, the clearance ($D_1$) of the hollow cavity between the top plate 500 and the sensor base plate 400 may be, for example, between 0.5 and 0.7 inches, or about 0.59 inches. The clearance ($D_2$) between the top surface (distal end) of the hard-stop rim 404 and the valley of the hard-stop recess 510 may be, for example, between 0.2 and 0.3 inches, or about 0.23 inches. The clearance ($D_3$) between the sensor base plate 400 and the bottom surface (distal end) of alignment lip 508 may be, for example, between 0.15 and 0.20 inches, or about 0.18 inches.

As illustrated, the interface bracket 222 is designed to non-invasively grip the exercise platform subassembly 104, while securely and physical coupling with the sensor base plate 400. Specifically, the attachment portion 222a of the interface bracket 222 may be fastened to the sensor base plate 400 via one or more fasteners 224. The fastener 224 may be a bolt, while the sensor base plate 400 may include a threaded bore sized and shaped to receive the threaded shaft of the fastener 224. The lip portion 222b is spaced from the fastener 224 such that the distanced ($D_4$) defined between the outer surface (bottom) of the sensor base plate 400 and the inner bottom surface 222c of the lip portion 222b is substantially the same as the thickness of the force plate 106. To further mitigate damage to the force plate 106, both inner contact surfaces of the interface bracket 222 (*i.e.,* the inner bottom surface 222c and the inner side surface 222d) may be padded, or otherwise lined, to avoid scratching the force plate 106, while also allowing for more play in the system and increasing friction between the interface bracket 222 and force plate 106. For example, the contact surfaces may include a layer of self-adhering silicone rubber. The self-adhering silicone rubber may be 1/6 inches thick by 1 inch wide and cut to the desired length.

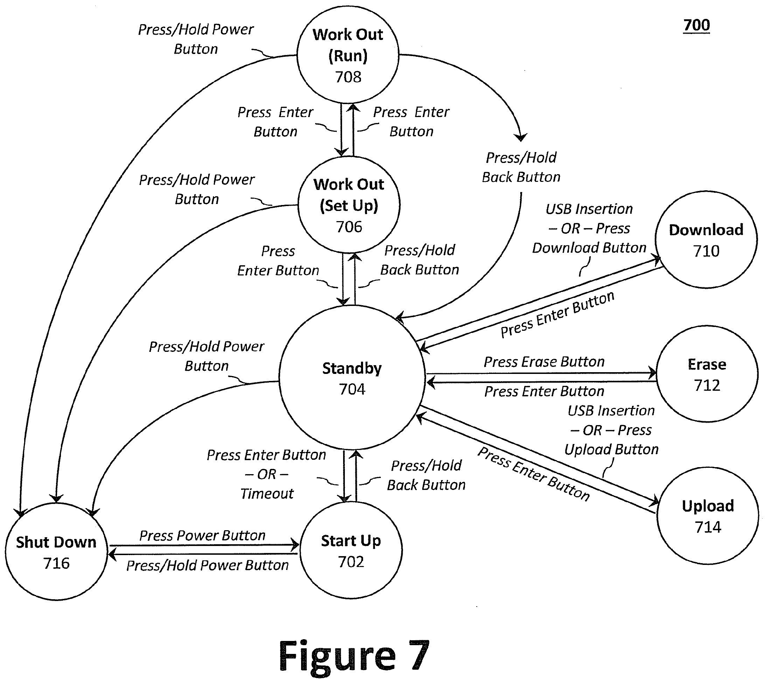

An objective of the dynamic load sensor system 200 is to offer on-orbit ease-of-use to crewmembers. Figure 7 illustrates an example process diagram 700 of the various operational modes. The operational modes of the system may include: a startup mode 702, a standby mode 704, a work out (set up) mode 706, a work out (run) mode 708, a download mode 712, an erase mode 714, an upload mode 710, and a shutdown mode 716.

The startup mode 702 is the first mode entered upon hardware power up (*e.g.,* power up of the ARED 100 or the dynamic load sensor system 200). The startup mode 702 may be triggered by switching on the physical power switch 238 at the processing unit 204, pressing a power button 218 (*e.g.,* at the operator interface module 206), or by pressing and holding the back button 214c on operator interface module 206 for a predetermined period of pressing time (*e.g.,* 2-10 seconds, more preferably about 3 seconds) while in another mode (*e.g.,* standby mode 704). During the startup mode 702, the dynamic load sensor system 200 performs a number of actions, including a hardware power up, a power-on-self-test (POST), a software boot-up, display of a splash screen (*e.g.,* "Aurora Flight Sciences" and copyright information), read INI file and prepare system for operation, and display a welcome screen (after boot-up). The welcome screen may provide the status (*e.g.,* ready, malfunction, etc.), the current date and time, the last log on date and time, and crew information (*e.g.,* crewmember ID). To enter the standby mode 704, the crewmember may actuate (*e.g.,* press) the enter button 214b on the operator interface module 206 or upon expiration of a timer. For example, if the crewmember is idle (*e.g.,* no input at the operator interface module 206 and/or lack of movement as sensed by the sensor pad unit 202) for a predetermined period of idle time (*e.g.,* 1 to 10 minutes, more preferably about 3 minutes), the dynamic load sensor system 200 may transition to the standby mode 704. Pressing and holding the power button 218 for the predetermined period of pressing time may causes the dynamic load sensor system 200 to transition to the shutdown mode 716.

The standby mode 704 is the mode during in which the dynamic load sensor system 200 awaits instruction from the crewmember. The standby mode 704 may be entered following startup mode 702, by pressing and holding the back button 214c, by pressing the enter button 214b during the download mode 712 or the upload mode 710, or by pressing the enter button 214b upon completion of the erase mode 714. During the standby mode 704, the dynamic load sensor system 200 displays an options screen (*e.g.,* workout, download data, erase internal files, upload initialization (INI) files, etc.), monitors the data ports 234 *(e.g.,* USB ports) for to detect a port insertion, and monitors the user input buttons (*e.g.*, function buttons 214). The crewmember can press the enter button 214b to select a default mode (*e.g.*, workout) or use the selector buttons 212 to navigate to a desired selection and then press the enter button 214b. Default selections can be highlighted on the operator interface module 206. Simply pressing the enter button 214b may selects a default mode and causes the dynamic load sensor system 200 to jump to the work out (set up) mode 706. When the dynamic load sensor system 200 detects a port insertion, the dynamic load sensor system 200 may jump to the download mode 712. In certain aspects, port insertion recognition may be limited to the only the standby mode 704, the download mode 712, and the upload mode 710. Pressing and holding the back button 214c may return to the standby mode 704, while pressing and holding power button 218 may jump to the shutdown mode 716. The work out (set up) mode 706 prepares the dynamic load sensor system 200 for data collection. The work out (set up) mode 706 may be entered from either the standby mode 704 or the work out (run) mode 708 by pressing the enter button 214b.

During the work out (set up) mode 706, the dynamic load sensor system 200 checks the amount of space available on Internal memory device 232. If there is insufficient disk space, the dynamic load sensor system 200 may display a warning screen (*e.g.,* "Warning: Insufficient Disk Space. Please Erase Old Files"). The warning screen may provide recommendation to erase files. To return to the standby mode 704, the crewmember may select the back button 214c. Otherwise, the crewmember may override the warning by selecting the enter button 214b, which will enable the crewmember to exercise, but data may not be recorded. Alternatively, the crewmember may elect to erase/overwrite files (*e.g.,* oldest data files) for the crewmember to create space for the new data.

The dynamic load sensor system 200 may also display a crewmember ID screen, which displays the crewmember names and/or ID numbers for the more recent crewmembers to use the ARED 100 or the dynamic load sensor system 200 (*e.g.,* the last 12 crewmembers). The crewmember may select his or her crewmember ID from the crewmember list, or simply his the enter button 214b to select a default crewmember ID. The crewmember ID screen may also enable the crewmember to enter a crewmember ID in a crewmember ID line if his or her crewmember ID is not included in the list display. To that end, the dynamic load sensor system 200 may display a keyboard (or a string of characters - letters/numbers) to allow the operator to select and input his or her crewmember ID. For example, the crewmember may use the selector buttons 212 to navigate to a desired character selection (the characters may be highlighted as the user moves through the list), whereupon pressing the enter button 214b places the highlighted character (*i.e.,* the desired character) into the crewmember ID line. The crewmember ID may default to "XXX" in the filename if nothing is entered.

Once the crewmember ID has been selected, the dynamic load sensor system 200 may display an exercise type screen, which displays a plurality of exercise options (*e.g.*, normal squat, single leg squat dead lift, etc.). The crewmember may simply select the enter button 214b to select a default exercise options. Alternatively, the crewmember may use the selector buttons 212 to navigate to a desired exercise option prior to pressing the enter button 214b.

Once the exercise option has been selected, the dynamic load sensor system 200 may display a confirmation screen, which may display confirmation data for the workout routine (*e.g.,* crewmember ID; workout type; force display units; force display measurements, etc.). To make changed to the workout routine, the crewmember may use selector buttons 212 to navigate and change a selection. For example, selecting the back button 214c while on the confirmation screen would return to the first step (*e.g.*, crewmember ID selection screen) to re-enter crewmember ID or change type of workout. Pressing and holding the back button 214c causes the dynamic load sensor system 200 to return to standby mode 704, while pressing and holding power button 218 causes the dynamic load sensor system 200 to jump to shutdown mode 716. To confirm the workout routine, the crewmember may select the enter button 214b, whereupon the dynamic load sensor system 200 enters the work out (run) mode 708. Pressing the enter button 214b while in the work out (run) mode 708 may enable the crewmember to change the type of workout.

During the work out (run) mode 708, the dynamic load sensor system 200 collects and records sensor data (*e.g.,* from the two sensor pad units 202). The work out (run) mode 708 may be entered by pressing the enter button 214b during work out (set up) mode 706. Further, the dynamic load sensor system 200 closes any open data files and displays a tare screen (which may also be called up via tare button 214a). The tare screen may display a message (*e.g.*, "Do Not Stand On Sensors"), while the dynamic load sensor system 200 zeros the load cells 248. Once the dynamic load sensor system 200 has zeroed the load cell data, it may begin to continuously collect sensor data in first in first out (FIFO) buffer. The dynamic load sensor system 200 may then open/create a data file with a predetermined filename format (*e.g.,* EDLS_<crewmember ID> VYYYMMDD_HHMMSS.dat), but does not yet write sensor data to the file. Upon creating the data file, the dynamic load sensor system 200 may display a run screen. The run screen may display, *inter alia,* the measured $F_z$, an identification of the units selected (*e.g.,* LBS, N, etc.), a load bar graph (min to max), and a status (*e.g.*, ready for workout, which may be the exercise option selected during the work out (set up) mode 706). The dynamic load sensor system 200 monitor the $F_z$ load values for threshold value to confirm that the device is still in use. The threshold value may be, for example, a nominal amount (*e.g.*, 1 to 10 pounds, more preferably about 2 pounds). For example, if $F_z >$ threshold value then record data in file on internal memory device 232, but if $F_z <$ threshold value for a predetermined time period (*e.g.,* 10 to 20 minutes, more preferably 15 minutes), then the process may restart (*i.e.,* return to the first step) by closing data file, re-zeroing the sensors, and opening new data file. In certain aspects, a crewmember can press and hold the tare button 214a to cause the dynamic load sensor system 200 to restart the work out (run) mode 708. To return to the work out (set up) mode 706, the crewmember may press the enter button 214b on the operator interface module 206.To return to the standby mode 704, the crewmember may press and hold the back button 214c. To enter the shutdown mode 716, the crewmember may press and hold the Power button 218. In certain aspects, the enter button 214b may be pressed in succession (*e.g.,* twice) to return to the work out (set up) mode 706.

During the download mode 712, the dynamic load sensor system 200 transfers (or copies) data file(s) from an internal memory device 232 of the dynamic load sensor system 200 to an external memory device 240. The download mode 712 may be automatically initiated when external memory device port insertion is detected during standby mode 704 or by selecting download data button 214f or via an options screen in standby mode 704. The dynamic load sensor system 200, during the download mode 712, may close any open data files, determines size of required transfer, and check for presence of external memory device 240. If an external memory device 240 is not found, the dynamic load sensor system 200 may display an error screen (*e.g.,* "Error - No External Memory Device Found" and/or "Please Insert External memory device 240"). In response, the crewmember may insert an external memory device 240 in a data ports 234 (*e.g.,* USB port) on operator interface module 206, or press the enter button 214b to return to the standby mode 704. Once an external memory device 240 is detected, the dynamic load sensor system 200 may check the external memory device 240 for sufficient space. If there is insufficient disk space, the dynamic load sensor system 200 may display a warning screen (*e.g.,* "Warning: Insufficient Disk Space Please Erase Old Files"). In response, the crewmember may press the enter button 214b to return to the standby mode 704. If there is sufficient space, the dynamic load sensor system 200 may start to download of all relevant data files on internal memory device 232 to the data ports 234 on the operator interface module 206, during which the operator interface module 206 may display a transfer screen (*e.g.,* the number of files to transfer, the percent transfer complete, which may be presented using a bar graph). Upon completion of file transfer, the operator interface module 206 may display a transfer complete screen (*e.g.,* "Transfer Complete: Okay to Remove External Memory Device"). After which, the crewmember may remove the external memory device 240 from the data port 234 and press the enter button 214b to return to the standby mode 704. Automatic port insertion recognition may be available during in the standby mode 704, download mode 712, and the upload mode 710.

The erase mode 714 enables the crewmember to erase data files from the dynamic load sensor system's 200 internal memory device 232. The erase mode 714 may be selected from the erase button 214d or an options screen in standby mode 704. During the erase mode 714, the dynamic load sensor system 200 may display a file erase screen (*e.g.,* number of files, percent erasure complete, which may be provided as a bar graph). To start erasing, the crewmember may select the enter button 214b. To mitigate inadvertent erasure, the dynamic load sensor system 200 may display an erasure confirmation screen (*e.g.,* "Are You Sure You Wish To Erase?"), whereupon erasure is started upon again pushing the enter button 214b. Upon completion of file erasure, the operator interface module 206 may display an erasure complete screen (*e.g.,* "Erasure Complete"). To return to the standby mode 704, the crewmember may press the enter button 214b.

The upload mode 710 enables the crewmember to transfer new initiation files from an external memory device 240 to the internal memory device 232. The upload mode 710 may be initiated by selecting the data upload button 214e or via an options screen in standby mode 704. During the upload mode 710, the dynamic load sensor system 200 checks for the presence of external memory device 240. If an external memory device 240 is not found, the dynamic load sensor system 200 may display an error screen (*e.g.*, "Error - No External Memory Device Found" and/or "Please Insert External Memory Device"). In response, the crewmember may insert an external memory device 240 in a port (*e.g.,* USB port) on operator interface module 206, or press the enter button 214b to return to the standby mode 704. If an external memory device 240 is detected, the dynamic load sensor system 200 checks for presence of predetermined files (*e.g.,* initiation (INI) files) on external memory device 240. If the predetermined files are not found, then the dynamic load sensor system 200 may display an error screen (*e.g.,* Error: No Files Found). After which, the crewmember may select the enter button 214b to return to Standby mode 704.

If one or more predetermined files are found, the interface module 206 may display, via display device 210, a transfer screen (*e.g.,* the number of files to transfer, percent transfer complete, which may be presented using a bar graph) and the predetermined file(s) may be uploaded from the external memory device 240 via the port to the internal memory device 232. upon completion of the file transfer, the interface module 206 may display a transfer complete screen (*e.g.,* "Transfer Complete: Okay to Remove External Memory Device"). After which, the crewmember may remove the external memory device 240 from the port and press the enter button 214b to return to the standby mode 704. As can be appreciated, the 200 may be configured to only upload the predetermined files from the external memory device 240, thereby ignoring any other files on external memory device 240. As noted above, automatic port insertion recognition may be available during in the standby mode 704, download mode 712, and the upload mode 710.

The shutdown mode 716 prepares the ARED 100 and/or the dynamic load sensor system 200 for removal of power. The shutdown mode 716 may be entered by pressing and holding the power button 218 during any of the startup mode 702, the standby mode 704, the work out (set up) mode 706, or the work out (run) mode 708. During the shutdown mode 716, the dynamic load sensor system 200 may close any open data files, display a shutdown status message (*e.g.*, "Preparing For Shutdown"), perform other shutdown preparations, start software shutdown, display a shutdown complete message (*e.g.*, "Safe To Remove Base Unit Power"), and finish software shutdown, to fully turn off the power supply to the dynamic load sensor system 200, a hardware shutdown may require switching a physical power switch 238 at the processing unit 204 to the "off position.

The dynamic load sensor system 200 employs unique techniques to achieve load sensor calibration, load measurement, load accuracy determination, and center-of-pressure calculation for the load sensors, extending the calibration routine to the load sensor corners, analysis code development and finite-element modeling to understand load sensor top plate bending to reduce edge and comer effects, and improving the accuracy and center-of-pressure determination outside of the internal load cell box.

The calibration routine is advantageous in that they extend to the edges of the top plate 500 and account for deflection (*i.e.,* bending) during loading near the edges and corners. Indeed, the calibration matrix incorporates a distinct calibration region outside of the load cell box 304 formed by the mounting locations of the four load cells 248, which accounts for the bending moments (about the load cells 248) that are present when a load is applied outside of the load cell box 304 (*see* Figure 3). The calibration algorithm accounts for the slight non-linear change in voltage values of each strain gauge of the load cells 248, which can be adjusted for different ranges of sensitivity depending on the magnitude of the applied force with three regions of sensitivity where the adjusted calibration values are used. This modified sensitivity is adjustable over different areas on the force plate (*i.e.,* the central load cell box as compared to the edge and corner regions).

In an effort to quantify the effects of torqueing upon the load cell fasteners has on the overall sensor sensitivity and hysteresis properties, each load cell 248 is considered separately prior to installation on the sensor base plate. Figure 6a shows the location of the four load cells 248 per sensor pad unit 202 with the sensor top plate 500 removed. An objective of the calibration procedure is to populate the calibration matrix [C], which is a collection of gains unique to each sensor pad unit 202 that maps the 6-axis forces and moments of interest to the strain gage voltages of the sensor pad unit's 202 load cells 248, such that:

$$\overline{F} = C\overline{V},$$

*Equation 1*

where $\overline{F}$ is a 6 element vector of orthogonal forces and moments, $C$ is the calibration matrix and $\overline{V}$ is the vector of strain gage voltages.

Each sensor pad unit 202 contains four load cells 248, each with 3 strain bridges, resulting in 12 strain gage voltages and a calibration matrix of size 6 x 12 with 72 unknown elements. The expression for the force-strain relation of the sensor pad unit 202 becomes:

$$\overline{F} = \begin{bmatrix} F_x \\ F_y \\ F_z \\ M_x \\ M_y \\ M_z \end{bmatrix}_{6 \times n} = \begin{bmatrix} c_{11} & c_{12} & \cdots & c_{1\,12} \\ c_{21} & & & \vdots \\ & & \ddots & \vdots \\ c_{61} & \cdots & \cdots & c_{6\,12} \end{bmatrix}_{6 \times 12} \begin{bmatrix} V_1 \\ V_2 \\ \vdots \\ \vdots \\ V_{11} \\ V_{12} \end{bmatrix}_{12 \times n}$$

*Equation 2*

A calibration matrix with 36 unknowns can be solved with linear algebra, however, the dynamic load sensor system 200 is statically indeterminate and the calibration matrix cannot be computed mathematically, but rather by measuring voltages for each of the 72 unique load cases and applying Least Squares Regression (LSR) techniques to identify the calibration coefficients $c_{11}$ through C6 12.

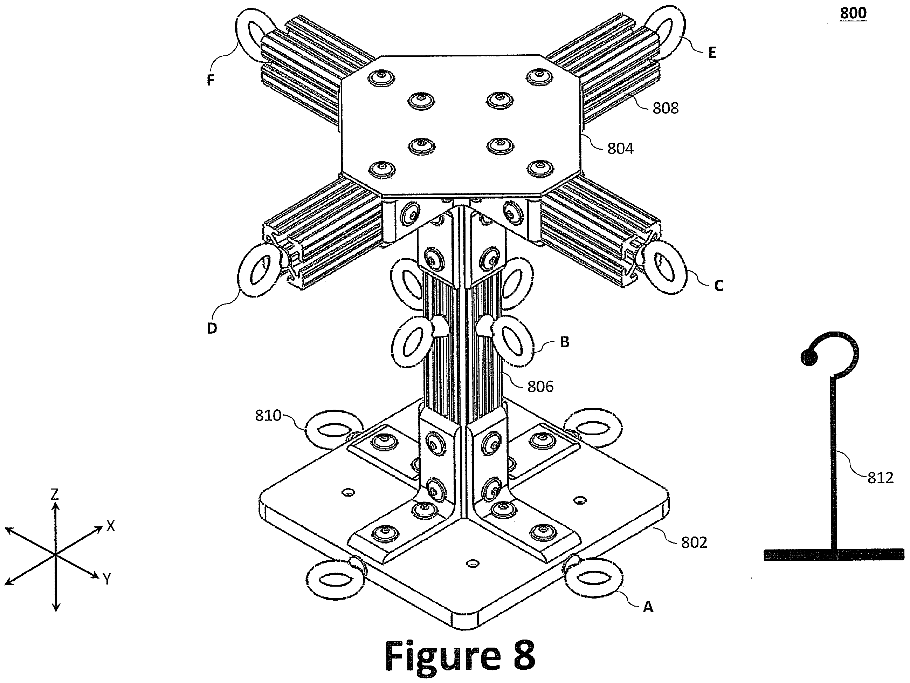

To achieve this, a set of known loads are applied to the sensor top plate through the use of a specialized calibration fixture 800, shown in Figure 8. As illustrated, the calibration fixture 800 generally comprises a lower (base) assembly 802 and an upper assembly 804 spaced from the lower assembly 802 via an intermediate support 806. The upper assembly 804 includes a plurality of booms 808 (*e.g.,* four) extending radially therefrom; each of the four booms 808 being positioned at right angles (90 degree) relative to its adjacent booms. The calibration fixture 800 allows for different combinations of forces and moments to be applied to the sensor pad unit 202 by hanging weights 812 off of a variety of eyebolts 810 located throughout the calibration fixture 800 (*e.g.,* locations A through F). For example, four eyebolts 810 may be provide on the upper assembly 804 (*e.g.,* one at the distal end of each boom 808), four eyebolts 810 may be provide on the intermediate support 806 (*e.g.,* on each sides of the intermediate support 806), and four eyebolts 810 may be provide on the lower assembly 802 (*e.g.,* on each sides of the lower assembly 802). The calibration fixture 800 may be fabricated using a light weight, high strength, and rigid component, which may be fabricated using metal extrusion techniques. Suitable metals include, for example, aluminum, brass, copper, lead (and tin), magnesium, zinc, steel, titanium, iron, and alloys thereof. Magnesium, whose material characteristics (*e.g.,* melting point) make it about as extrudable as aluminum, is particular well suited for aircraft parts and nuclear industry parts. For example, as illustrated, the booms 808 and the intermediate support 806 may be fabricated as T-slot extruded bars. By way of illustration, the empty calibration fixture 800 weighs 6.0 kg (13.2 lbs), the associated weight hanger that hooks into its eyebolts 810 weighs 1.3 kg (3.0 lbs), and the top plate 500 weighs 3.0 kg (6.5 lbs). As noted above, however, the top plate 500 may weigh more if the triangular pattern is omitted.

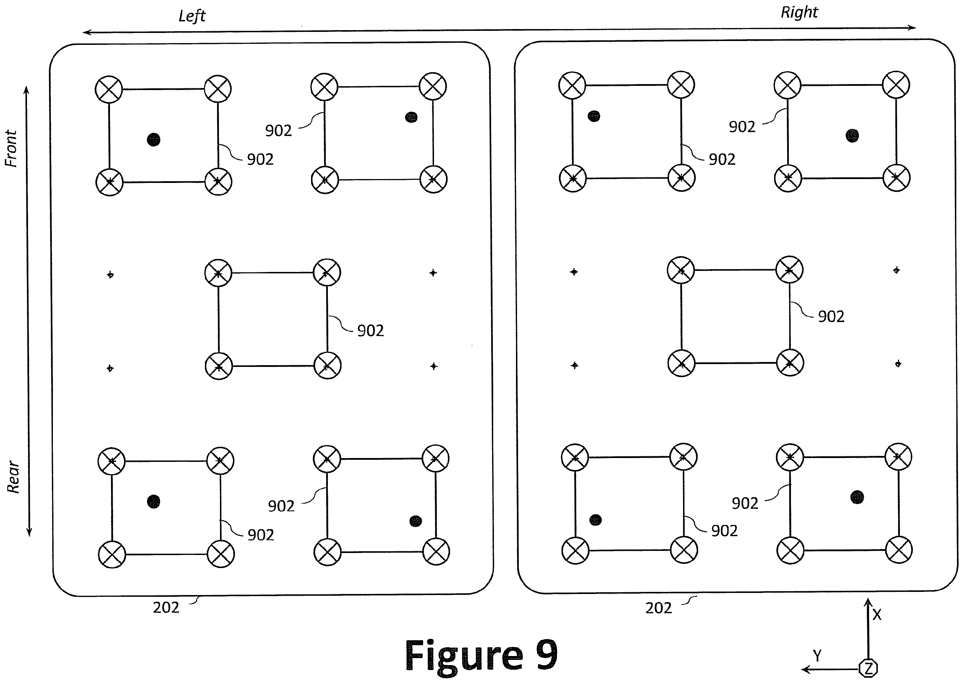

During calibration, the calibration fixture 800 is attached to the sensor pad unit 202 via its lower assembly 802 with an independent set of screws to ensure that the torque of the screws that secure the sensor top plate to the load cells 248 remains unchanged. The calibration fixture 800 is designed to mount at different locations on the sensor top plate to produce unique loading profiles for identifying the 72 elements of the calibration matrix. Figure 9 illustrates a planar view of a dimensioned sensor pad unit 202 with the five different fixture mounting locations 902 in a defined coordinate system. Note that a right handed coordinate system is used in connection with certain of the figures, including Figure 9.

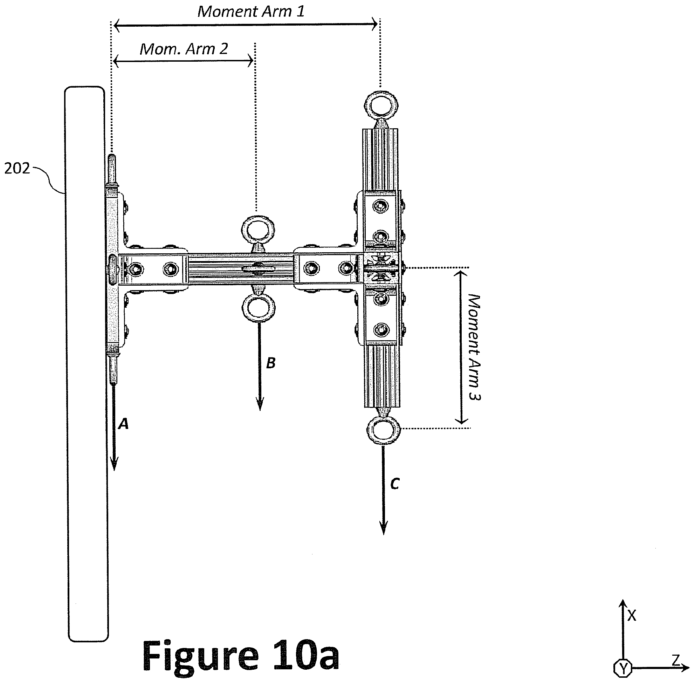

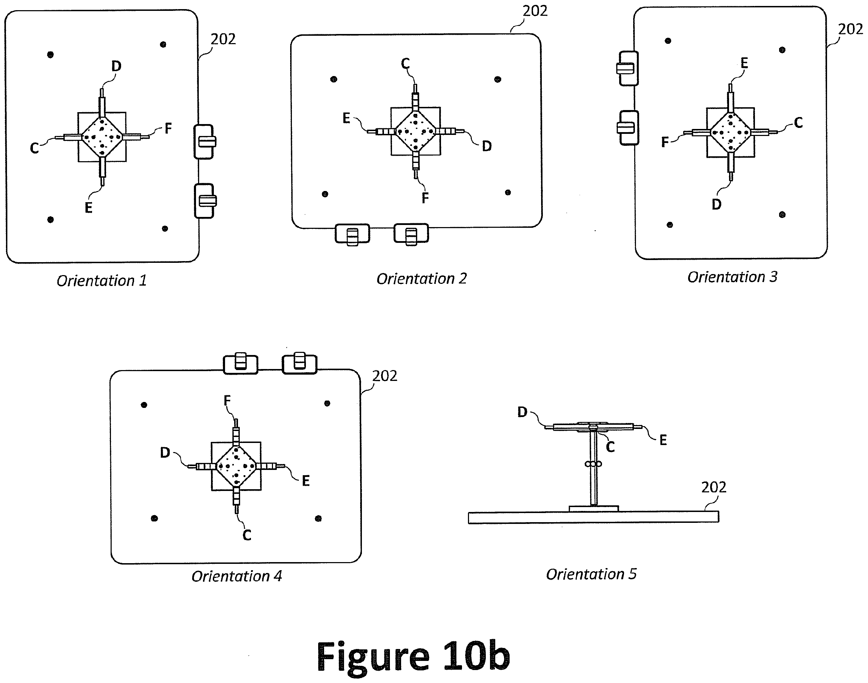

With reference to Figures 10a and 10b, during calibration, the sensor pad unit 202 is fitted with the calibration fixture 800 and is mounted vertically, so that gravity acts singularly in the direction of the +x, -x, +y or -y axis. The assembly (*i.e.,* the sensor pad unit 202 fitted with the calibration fixture 800) is rotated about the z-axis as illustrated in Figure 10b (orientations 1 through 4) to allow for a different axis to point towards the ground, aligning it with the load path. In each orientation, hanging weights 812 are hung at various moment arm locations to produce known moments, as best shown in Figure 10a. For example, Figure 10a illustrates the calibration fixture 800 mounted vertically with load locations A, B and C shown. The length of moment arm 1 may be, for example, 17.97 cm (7.08 in), while lengths of moment arms 2 and 3 may be, respectively, 31.31 cm (12.33 in) and 19.37 cm (7.63 in). All z-axis loads are applied by loading weights on the top center of the calibration fixture 800 (*i.e.,* on top of the upper assembly 804) with the sensor pad unit 202 placed on a flat surface, *i.e.,* perpendicular to the gravity vector. An example of which is illustrated in Figure 10b as orientation 5.

The calibration fixture 800 is designed such that it does not require reorientation when a different sensor axis is aligned with the gravity vector. Furthermore, the calibration fixture 800 and sensor pad unit 202 assembly is mounted to a vertical turntable when loaded, allowing it to rotate so that each of the major x- and y-axes aligns with the gravity vector within ±1 degree, as shown in Table 1. This approach minimizes sensor handling during calibration, and so limits hysteresis effects that may alter zero-load sensor readings. After each rotation, the assembly's perpendicularity relative to gravity is confirmed with a spirit level and the plate secured to prevent any rotation during calibration. Care should also be taken to develop a turntable of adequate stiffness and so prevent flexing of the turntable during loading.

Table 1, with reference to Figure 10b, shows the orientation of the assembly during each load case as well as the associated known forces and moments, while Figure 6 best illustrated the loading locations (*e.g.,* loading locations A through F).

*Table 1*

| Orientation (See Figure 10b) | Direction of Gravity | Loading Location | Applied Forces and Moments |
|---|---|---|---|
| Orientation 1 | -x | A | -Fx |
| | -x | B | -Fx, +My |
| | -x | C | -Fx, +My |
| | -x | D | -Fx, +My, -Mz |
| | -x | E | -Fx, +My, +Mz |
| Orientation 2 | +y | A | +Fy |
| | +y | B | +Fy, +Mx |
| | +y | C | +Fy, +Mx |
| | +y | D | +Fy, +Mx, -Mz |
| | +y | E | +Fy, +Mx, +Mz |
| Orientation 3 | +x | A | +Fx |
| | +x | B | +Fx, -My |
| | +x | C | +Fx, -My |
| | +x | D | +Fx, -My, -Mz |
| | +x | E | +Fx, -My, +Mz |
| Orientation 4 | -y | A | -Fy |
| | -y | B | -Fy, -Mx |
| | -y | C | -Fy, -Mx |
| | -y | D | -Fy, -Mx, -Mz |
| | -y | E | -Fy, -Mx, +Mz |
| Orientation 5 | +z | - | +Fz |
| | +z | C | +Fz, -Mx |
| | +z | F | +Fz, +Mx |
| | +z | D | +Fz, -My |
| | +z | E | +Fz, +My |

For each of orientations 1 through 4, the assembly is successively loaded with 2 lb (0.9 kg) weight increments so that the load increases from 0 to 20 lbs (9.1 kg) and decreases in a similar fashion to capture any hysteresis effects: $0 \to 2 \to 4 \to 6 \to 8 \to 10 \to 12 \to 14 \to 16 \to 18 \to 20 \to 18 \to 16 \to 14 \to 12 \to 10 \to 8 \to 6 \to 4 \to 2 \to 0$. In the case of the +z axis loading (orientation 5) where the sensor pad unit 202 is perpendicular to the gravity vector, the load is increased from 20 to 400 lbs in 20 lb increments, as this is the load direction of least interest. Small loads less than 20 lbs are of interest as data suggests that over 90% of adaptation motions occur in this range. From 20 lbs and higher, the calibration increment increases to 20 lb as such fine resolution is less critical. Given these 21 load cases per orientation, and a total of 5 orientations, each with 5 loading locations as per Table 1, a total $n$ of 525 load cases will be recorded per sensor pad unit 202. Data is recorded at 250 Hz and averaged over a 3 second period, with sufficient time for transient responses to subside.

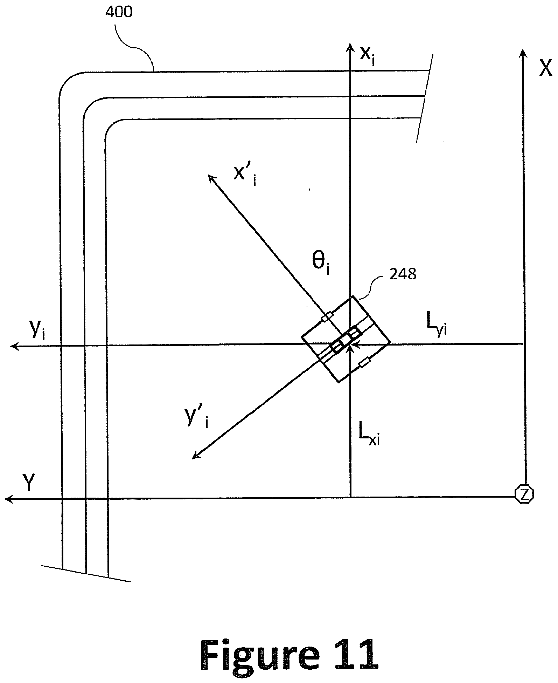

In addition to the sensor pad unit 202 calibration, each load cell 248 is calibrated individually and the local calibration coefficients converted to global reaction forces and moments. The calibration matrix converts voltages ($v_{xi}$, $v_{yi}$, and $v_{zi}$, collectively, $V$) from individual load cells 248 to a global reaction forces and moments ($f_{Gx}$, $f_{Gy}$, $f_{Gz}$, $m_{Gx}$, and $m_{Gz}$, collectively, $F_G$). The approach may be broken down into the following high level steps. First, determine the calibration coefficients [C], which converts local load cell voltages to local reaction forces. Second, determine the rotation matrix [R], which transforms load cell forces from local coordinates to global coordinates (dependent on the location rotation angles, $\theta_i$). Third, determined the summation matrix [A], which sums local forces (in global coordinates) to global reaction forces and moments (dependent on local position on each load cell, ($x_i$, $y_i$). The coordinate systems and load cell 248 positioning variables are shown in Figure 11. The total calibration matrix, [G] may be given by:

$$[F_G] = [G][V],$$

*Equation 3*

where

$$[G] = [A][R][C].$$

*Equation 4*

The local calibration matrices, $[c]i$, which convert local voltages to local forces (*i.e.,* $f'_{xi}$, $f'_{yi}$ and $f'_{zi}$, collectively $F'_i$), must be determined experimentally for each load cell and is given by:

$$\underbrace{\begin{bmatrix} f'_x \\ f'_y \\ f'_z \end{bmatrix}_i}_{[F']_i} = \underbrace{\begin{bmatrix} c_{i11} & c_{i12} & c_{i13} \\ c_{i21} & c_{i22} & c_{i23} \\ c_{i31} & c_{i32} & c_{i33} \end{bmatrix}}_{[c_i]} \underbrace{\begin{bmatrix} v_{xi} \\ v_{yi} \\ v_{zi} \end{bmatrix}_i}_{[V]_i}$$

*Equation 5*

These local calibration matrices are arranged into a global calibration matrix, $[C]$, such that the local forces $F'_i$, are related to the measured voltages, $V_i$, in the relation:

$$\begin{bmatrix} [F']_1 \\ [F']_2 \\ [F']_3 \\ [F']_4 \end{bmatrix} = \underbrace{\begin{bmatrix} c_1 & 0 & \cdots & 0 \\ 0 & c_3 & \ddots & \vdots \\ \vdots & \ddots & c_3 & 0 \\ 0 & \cdots & 0 & c_4 \end{bmatrix}}_{[C]} \begin{bmatrix} [V]_1 \\ [V]_2 \\ [V]_3 \\ [V]_4 \end{bmatrix}$$

*Equation 6*

The local forces, $F'_i$, are measured in local coordinate systems and must be rotated to align with global coordinates. The globally aligned local forces are denoted without the apostrophe (') as $F_i$. A local rotation matrix, $R_i$, is dependent on the load cell 248 rotation angle, $\theta_i$, and is given by:

$$\underbrace{\begin{bmatrix} f_{xi} \\ f_{yi} \\ f_{zi} \end{bmatrix}}_{[F]_i} = \underbrace{\begin{bmatrix} \cos\theta_i & -\sin\theta_i & 0 \\ \sin\theta_i & \cos\theta_i & 0 \\ 0 & 0 & 1 \end{bmatrix}}_{[R_i]} \begin{bmatrix} f'_{xi} \\ f'_{yi} \\ f'_{zi} \end{bmatrix}$$

*Equation 7*

The local rotation matrices, $R_i$, are arranged into a global rotation matrix, $[R]$:

$$\begin{bmatrix} [F]_1 \\ [F]_2 \\ [F]_3 \\ [F]_4 \end{bmatrix} = \underbrace{\begin{bmatrix} R_1 & 0 & \cdots & 0 \\ 0 & R_3 & \ddots & \vdots \\ \vdots & \ddots & R_3 & 0 \\ 0 & \cdots & 0 & R_4 \end{bmatrix}}_{[R]} \begin{bmatrix} [F']_1 \\ [F']_2 \\ [F']_3 \\ [F']_4 \end{bmatrix}.$$

*Equation 8*

With the local load cell reaction forces aligned in global coordinates, the global reaction forces and moments applied to the sensor can be determined. The forces applied to the sensors are a sum of the local load cell reaction forces in each of the orthogonal directions. The moments applied to the sensor are calculated by considering the torque about the reference axis being contributed by each load cells 248 (forces * distance). The contribution to global force of each cell, $F_{Gi}$, is given as:

$$\underbrace{\begin{bmatrix} f_{Gxi} \\ f_{Gyi} \\ f_{Gzi} \\ m_{Gxi} \\ m_{Gyi} \\ m_{Gzi} \end{bmatrix}}_{[F_G]_i} = \underbrace{\begin{bmatrix} 1 & 0 & 0 \\ 0 & 1 & 0 \\ 0 & 0 & 1 \\ 0 & 0 & L_{yi} \\ 0 & 0 & -L_{xi} \\ -L_{yi} & L_{xi} & 0 \end{bmatrix}}_{[A]_i} \begin{bmatrix} f_{xi} \\ f_{yi} \\ f_{zi} \end{bmatrix}$$

*Equation 9*

Due to superposition, the total global forces can be written simply as the sum of contribution from each of the load cells 248:

$$[F_G] = \underbrace{\begin{bmatrix} [A]_1 & [A]_2 & [A]_3 & [A]_4 \end{bmatrix}}_{[A]} \begin{bmatrix} [F]_1 \\ [F]_2 \\ [F]_3 \\ [F]_4 \end{bmatrix}$$

*Equation 10*

In view of the forgoing, the matric expression that converts local load cell voltages to a global reaction forces can therefore be expressed mathematically as:

$$F_G = [A][R][C][V],$$

*Equation 11*

where $F_G$ is the global forces and moments vector, $A$ is the summation matrix that sums local forces in global coordinates, $R$ is the rotation matrix, C is the load cell calibration coefficient matrix, and $V$ is the measured voltage vector.

The load cell 248 calibration procedure may be employed to verify that no significant stress contribution is introduced after the load cells 248 are installed in the sensor pad unit 202. Prior to installation on the sensor pad unit 202 each load cell 248 is suspended and loaded in all major axes in a similar fashion to the sensor pad unit 202, except that the maximum load is reduced to 5 lbs as the 20 lbs load is distributed over four load cells 248 so that the load profile follows: $0 \rightarrow 1 \rightarrow 2 \rightarrow 3 \rightarrow 4 \rightarrow 5 \rightarrow 4 \rightarrow 3 \rightarrow 2 \rightarrow 1 \rightarrow 0$.

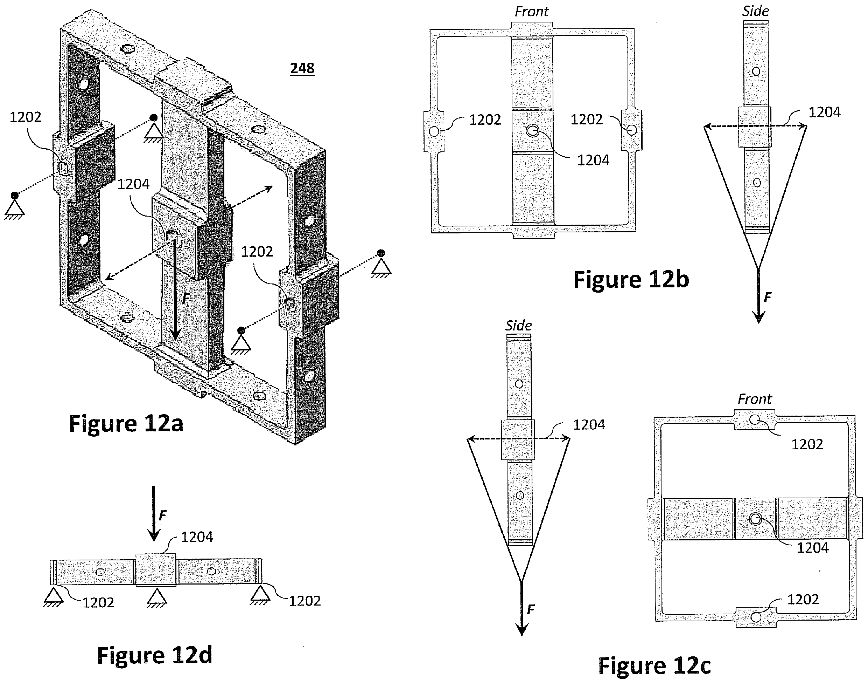

Figures 12a through 12d show the various orientations of the load cells 248 during the load cell 248 calibration procedure. In certain views, a dashed line is used to depict the force location (*e.g.,* center hole 1204) where the force is applied, while the support areas (*e.g.,* lateral holes 1202) where the load cell 248 is supported are annotated using dotted lines. In each of the figures, the force vector (F) is represented by a black arrow. In parallel loading (+x, -x, +y, -y axes) the load cell 248 is suspended and supported via the lateral holes 1202, while the force is applied via a rod passing through the center hole 1204. Loading via the rod ensures no additional z plane moments are present during calibration. Figure 12b illustrates front and side views of load cell 248, orientated for loading in the y-axis. The side view shows the location where the force is applied, corresponding with the center of gravity the load cell 248, resulting in zero z-plane moments. Figure 12c illustrates front and side views of the load cell 248 suspended with x-axis aligned with the gravity vector. In the case of z-axis loading, the load cell 248 is positioned perpendicular to the gravity vector and the load applied by positioning weight on the square area in the center of the load cell 248. Specifically, Figure 12d illustrates a force applied perpendicularly to the load cell plane (along z-axis). Similar to the case of the sensor assembly calibration, the load in the z-axis is incrementally increased to 100 lbs to resemble loads expected during use on orbit.

Once calibration is completed, a validation test can be performed. For example, controlled loads ranging from 0 to 400 lbs (181.4 kg) can be applied in increments of 15.0 lbs (6.8 kg), applied every 3.0 inches (7.6 cm) throughout the top plate of the sensor. The loads can be ramped up and back down to assess hysteresis effects. This test may be used to verify load accuracies and to calculate the associated center of pressure for each sensor pad unit 202.

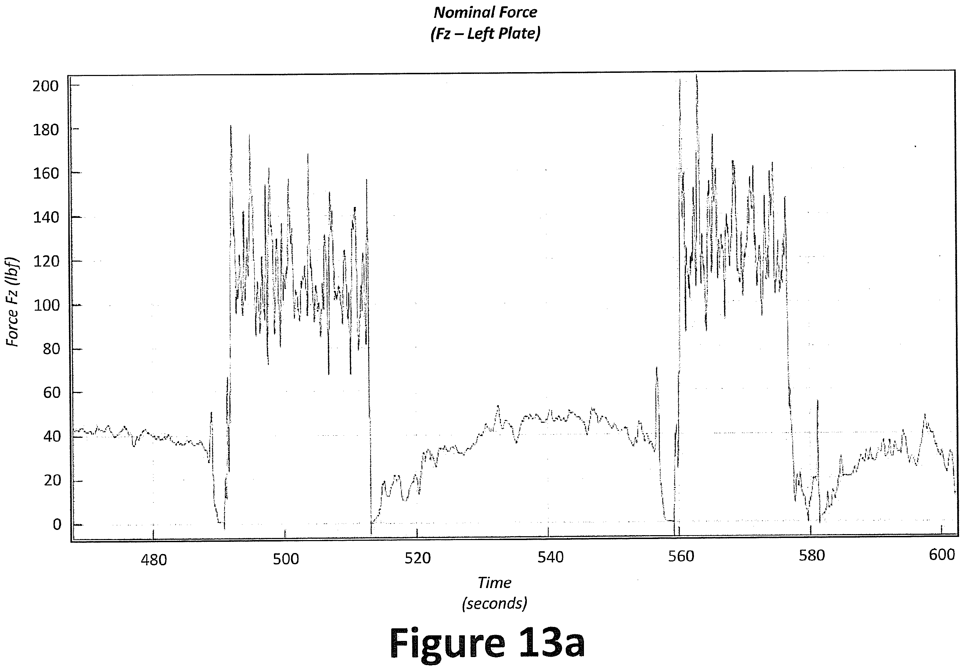

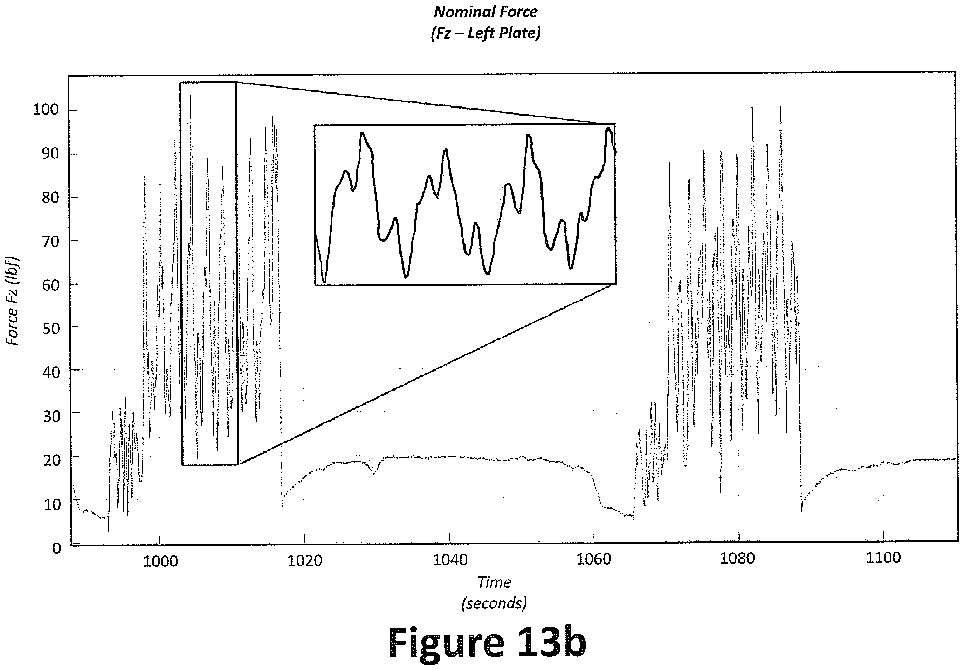

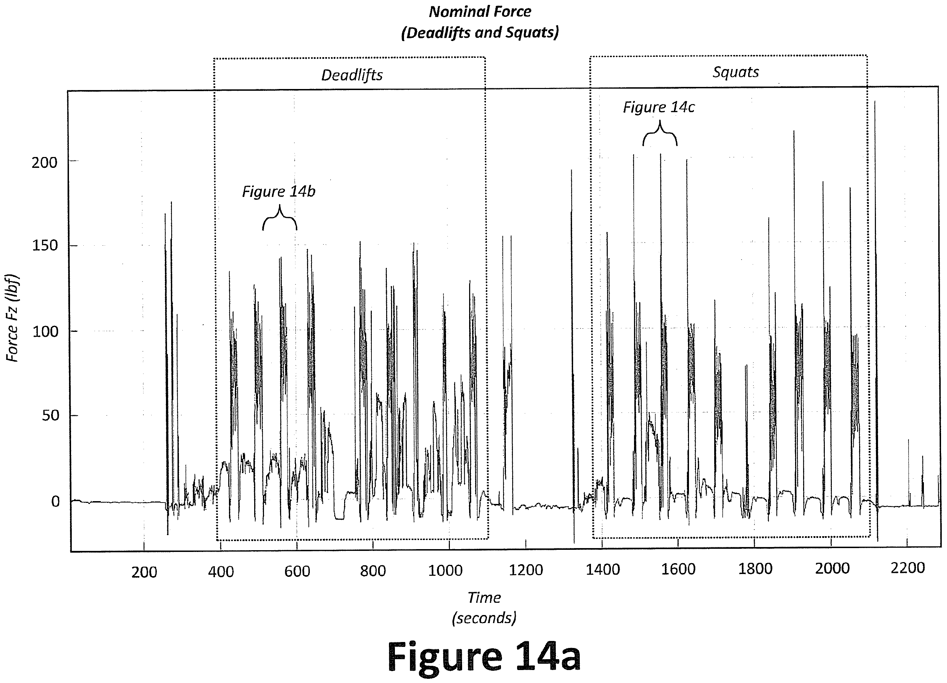

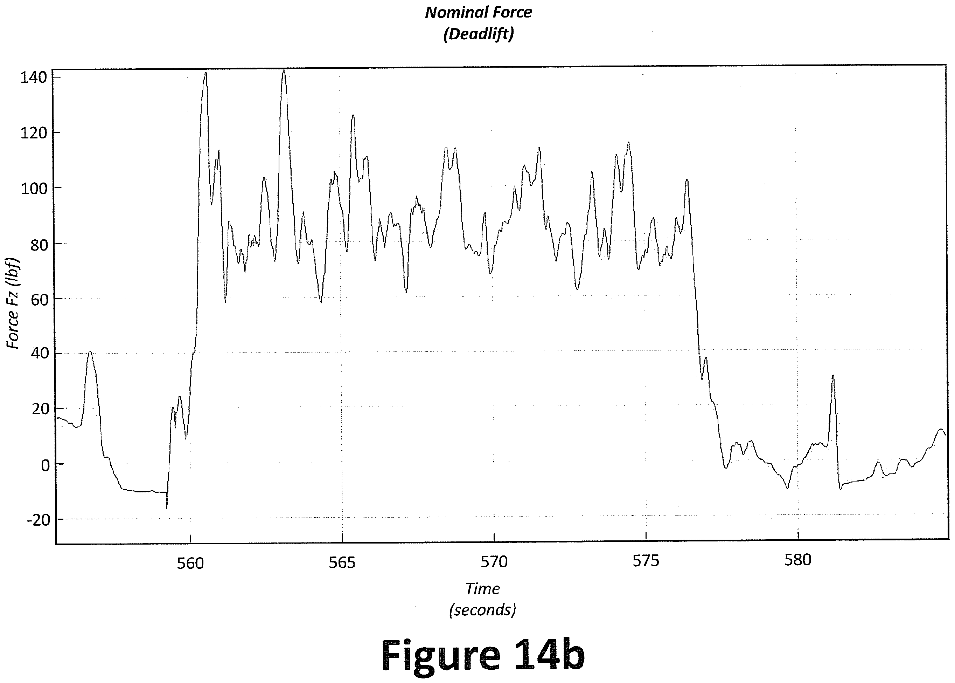

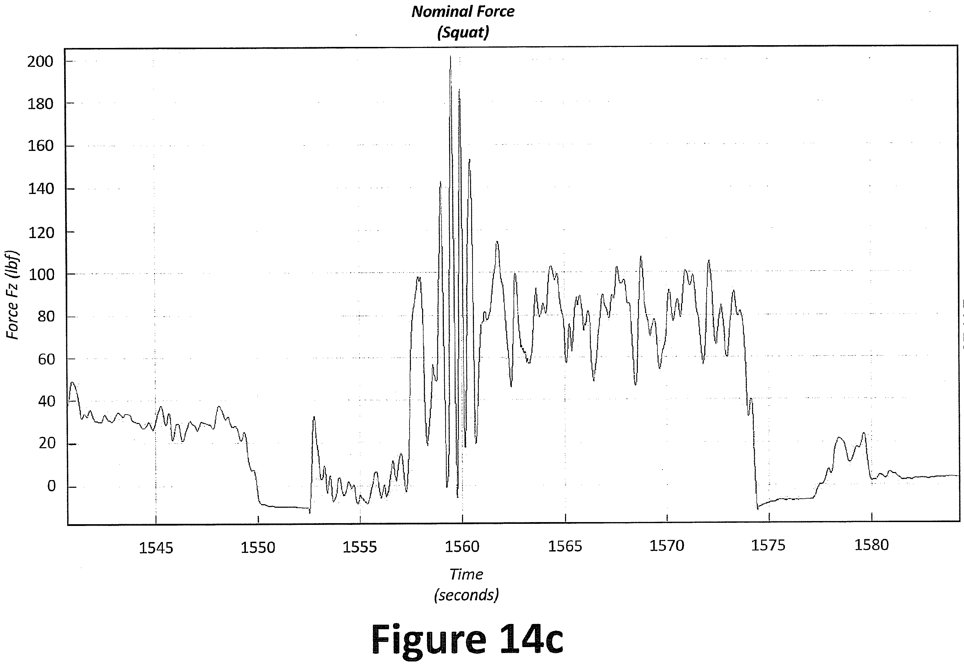

Parabolic flight testing was performed to test the hardware of the dynamic load sensor system 200 and to calibrate the HILT data during 160 parabolas (112 at zero gravity (0 g), 48 at lunar gravity (1/6 g)). Figures 13a and 13b illustrate example deadlift and squat data collected during the parabolic flights. A comparison to on orbit and other ground-based systems indicate that these results successfully replicate what is expected during reduced gravity loading. Figure 14a illustrates additional sensor unit data during parabolic flight testing, where Figures 14b and 14c illustrate enlargements of, respectively, the deadlift and squat exercise routines.

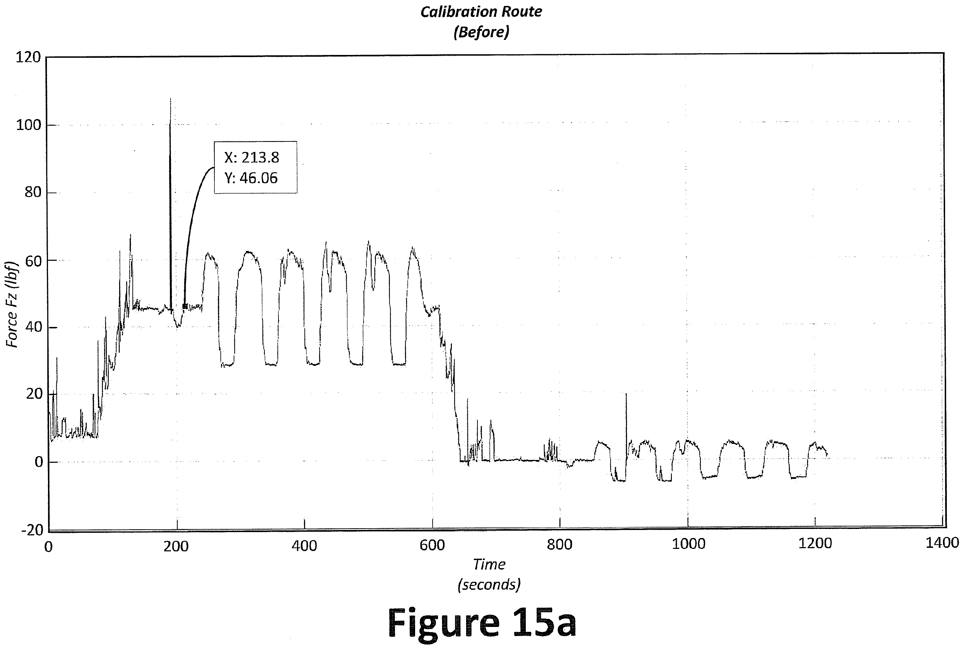

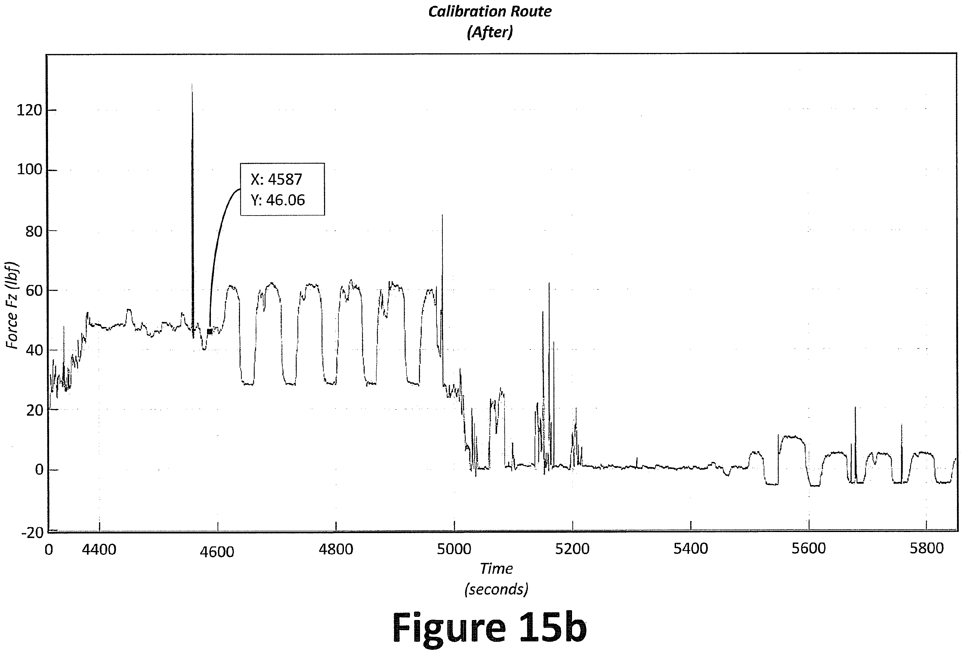

Figures 15a and 15b illustrate graphs of the data collected during the static load calibration verification test conducted at the start and end of a parabolic flight testing campaign. As can be seen from the data, the average 1g, 0g and 2g load cases remained consistent with an error within 1% of full scale load throughout the campaign. These results are particularly promising since an unplanned impulse loading incident did not affect the calibration of the sensors and verifies that the system is robust enough to accommodate impact loading. While performing heavy deadlift exercises (280 lbs weight) during one of the parabolic flights, the bar slipped from the subject's hand and, while not causing a safety hazard, impacted the sensor pad units 202 at high velocity. The impact, while unplanned, aided in proving sensor robustness since it did not affect the calibration, validating the design for use on International Space Station. This is notable because excessive loading can occur during launch to International Space Station.

While the dynamic load sensor system 200 has been described primarily with regard to use in microgravity environments, the present disclosure should not be construed as limited to use in microgravity environment. Rather, the teachings of the present disclosure may be applied to Earth-based (*i.e.,* gravity-based) exercise equipment for use in high-performance sports training (*e.g.,* professional sports, the Olympics); research/laboratory testing requiring posture, limb, or body kinetic information (*e.g.,* prosthetic development, vestibular research, microgravity adaptation).

The above-cited patents and patent publications are hereby incorporated by reference in their entirety. Although various embodiments have been described with reference to a particular arrangement of parts, features, and like, these are not intended to exhaust all possible arrangements or features, and indeed many other embodiments, modifications, and variations may be ascertainable to those of skill in the art. Thus, it is to be understood that the invention may therefore be practiced otherwise than as specifically described above.

## Claims

1. A dynamic load sensor system (200) for use in microgravity environments, the dynamic load sensor system (200) comprising:

   a first sensor pad unit (202) and a second sensor pad unit (202), wherein each of the first sensor pad unit (202) and the second sensor pad unit (202) comprises a sensor base plate (400) (400), a top plate (500), and a plurality of load cells (248) positioned between the sensor base plate (400) and the top plate (500);
   a processing unit (204) having a processor (228) operatively coupled with an internal memory device (232); and
   an operator interface module (206) having a display device and a plurality of operator input devices (242).

2. The dynamic load sensor system (200) of claim 1, wherein each of the plurality of load cells (248) comprises twelve foil strain gages.

3. The dynamic load sensor system (200) of claim 1 or 2, wherein each of the plurality of load cells (248) comprises three full Wheatstone strain gage bridges.

4. The dynamic load sensor system (200) of any of claims 1 to 3 wherein the operator interface module (206) enables an operator to navigate between a plurality of operational modes using one or more of the plurality of operator input devices (242).

5. The dynamic load sensor system (200) of any of claims 1 to 4 wherein each of the first sensor pad unit (202) and the second sensor pad unit (202) are configured to non-invasively couple with a preexisting exercise device.

6. The dynamic load sensor system (200) of any of claims 1 to 5, wherein each of the processing unit (204), the operator interface module, the first sensor pad unit (202), and the second sensor pad unit (202) are separate components, and the processing unit (204) is operatively coupled with the operator interface module (206), the first sensor pad unit (202), and the second sensor pad unit (202) via a plurality of interconnecting cables.

7. The dynamic load sensor system (200) of any of claims 1 to 6, wherein at least one of the processing unit (204) or the operator interface module (206) includes a data port communicatively coupled with the processor (228), the data port being configured to removably couple with an external memory device (240).

8. The dynamic load sensor system (200) of any of claims 1 to 7 wherein the top plate (500) comprises a first surface residing in a first plane and a second surface residing in a second plane that is substantially parallel to the first plane, wherein the first surface is shaped to include a geometric pattern.

9. The dynamic load sensor system (200) of any of claims 1 to 8 wherein the sensor base plate (400) comprises a first surface residing in a first plane and a second surface residing in a second plane that is substantially parallel to the first plane, wherein the first surface includes a plurality of sensor recesses to secure the plurality of load cell (248)s.

10. A method for performing load sensor calibration of a sensor pad unit (202) in a dynamic load sensor system, the sensor pad unit (202) having a plurality of load cell (248)s positioned between a sensor base plate (400) and a top plate (500), the method comprising:

   determining a first calibration coefficient for a first load cell (248) having a first rotation angle, and a second calibration coefficient for a second load cell (248) having a second rotation angle;
   converting a first local load cell (248) voltage from the first load cell (248) to a first local reaction force using the first calibration coefficient, and a second local load cell (248) voltage from the second load cell (248) to a second local reaction force using the second calibration coefficient;
   determining a first rotation matrix for the first load cell (248) as a function of the first rotation angle, and a second rotation matrix for the second load cell (248) as a function of the second rotation angle;
   transforming the first local reaction force from a local coordinate to a global coordinate as a function of the first rotation matrix to yield a first global reaction force, and the second local reaction force from a local coordinate to a global coordinate as a function of the second rotation matrix to yield a second global reaction force;
   determining a summation matrix to sum the first global reaction force and the second global reaction force; and summing the first global reaction force and the second global reaction force using the summation matrix to provide global reaction forces and moments for the sensor pad unit (202).

11. The method of claim 10, further comprising the steps of:

   determining a third calibration coefficient for a third load cell (248) having a third rotation angle, and a fourth calibration coefficient for a fourth load cell (248) having a fourth rotation angle;
   converting a third local load cell (248) voltage from the third load cell (248) to a third local reaction force using the third calibration coefficient, and a fourth local load cell (248) voltage from the fourth load cell (248) to a fourth local reaction force using the fourth calibration coefficient;
   determining a third rotation matrix for the third load cell (248) as a function of the third rotation angle, and a fourth rotation matrix for the fourth load cell (248) as a function of the fourth rotation angle; and
   transforming the third local reaction force from a local coordinate to a global coordinate as a function of the third rotation matrix to yield a third global reaction force, and the fourth local reaction force from a local coordinate to a global coordinate as a function of the fourth rotation matrix to yield a fourth global reaction force, wherein the global reaction forces and moments for the sensor pad unit (202) is a summation of the first, second, third, and fourth global reaction forces.

12. The method of claim 11, wherein each of the first, second, third, and fourth calibration coefficients is determined experimentally.

13. The method of claim 12, wherein each of the plurality of load cells (248) comprises twelve foil strain gages.

14. The method of claim 12, wherein each of the plurality of load cells (248) comprises three full Wheatstone strain gage bridges.

**Figure 1a**

EP 3 441 738 A2

## Figure 1b

**Figure 2a**

EP 3 441 738 A2

**Figure 2b**

EP 3 441 738 A2

**Figure 2c**

**Figure 3**

**Figure 4**

**Figure 5a**

EP 3 441 738 A2

**Figure 5b**

EP 3 441 738 A2

**Figure 6a**

EP 3 441 738 A2

Figure 6b

**Figure 6c**

EP 3 441 738 A2

**Figure 7**

EP 3 441 738 A2

Figure 8

Figure 9

EP 3 441 738 A2

Figure 10a

EP 3 441 738 A2

Orientation 1

Orientation 2

Orientation 3

Orientation 4

Orientation 5

**Figure 10b**

EP 3 441 738 A2

**Figure 11**

EP 3 441 738 A2

Figure 12b

Figure 12c

Figure 12a

Figure 12d

42

Figure 13a

EP 3 441 738 A2

## Nominal Force
## (Fz – Left Plate)

Force Fz (lbf)

Time
(seconds)

# Figure 13b

EP 3 441 738 A2

EP 3 441 738 A2

**Nominal Force**
*(Deadlifts and Squats)*

Figure 14a

**Nominal Force**
*(Deadlift)*

**Figure 14b**

**Nominal Force
(Squat)**

Time
(seconds)

# Figure 14c

**Figure 15a**

EP 3 441 738 A2

**Figure 15b**